# EUROPEAN PATENT APPLICATION

(11) **EP 1 790 983 A1**
(43) Date of publication of application: **30.05.2007**
(21) Application number: 05768836.8
(22) Date of filing: 29.07.2005
(51) Int. Cl.: G01N 33/50, A61K 45/00, A61P 1/16, A61P 31/12, C12Q 1/02, G01N 33/15, G01N 33/566, C12N 15/09

(54) **METHOD OF SCREENING COMPOUND PREVENTING CELLS FROM INFECTION WITH HEPATITIS C VIRUS (HCV)**

(30) Priority: 30.07.2004 JP 2004224741; 27.08.2004 US 605298 P; 01.10.2004 JP 2004290106; 13.10.2004 US 618238 P
(71) Applicant: Japan Tobacco, Inc., Tokyo 105-8422 (JP); Matsuura, Yoshiharu, Osaka 565-0821 (JP); Lim, Chang, Kwang, Tokyo 183-0052 (JP)
(72) Inventor: MATSUURA, Yoshiharu, Suita-shi, Osaka 565-0821 (JP); LIM, Chang, kwang, ; 2-503, Fuchu dai2 jutaku, Fuchu-shi, Tokyo 183-0052 (JP); KOMODA, Y.; c/o Central Pharm. Res. Inst. of Japan, Osaka 569-1125 (JP); SUZUKI, K. c/o Pharm. Frontier Research Lab. of, Yokohama-shi, Kanagawa 2360-004 (JP)
(74) Representative: Vossius & Partner
(86) International application number: PCT/JP2005/014374
(87) International publication number: WO 2006/011686

(57) **Abstract**

The present invention relates to a screening method and identification method for a compound that inhibits the cell infection of hepatitis C virus (HCV), which comprises measuring affinity of a test compound for fibroblast growth factor receptor (FGFR) or the capability of blocking the binding thereof to HCV, and selecting or judging a test compound.

## Description

### Technical Field

The present invention relates to a screening method and identification method for a compound that inhibits the cell infection of hepatitis C virus (HCV) by a novel action mechanism. The present invention also relates to an inhibitor of the cell infection of the virus comprising a compound that inhibits the cell infection of HCV, that can be obtained by this screening method or identification method.

### Background Art

In 1989, a major causative virus for non-A non-B hepatitis of post-blood transfusion was discovered and designated as hepatitis C virus (HCV). At present, in addition to type A, type B, and type C, several kinds of hepatitis viruses have been discovered, and the hepatitis caused by HCV is called hepatitis C. HCV-infected patients are estimated to account for as many as several percent of the population of the entire world, and the infection is characterized by chronic prolongation. Hepatitis C is also Japan's national disease; there is a strong demand for the establishment of a method for preventing the onset in the carrier and aggressively eliminating the virus from the body.

HCV is an RNA virus having an envelope, with its genome being plus single-stranded RNA, and is classified under the Hepacivirus genus in the family *Flaviridae* (as classified by The International Committee on Taxonomy of Viruses of the International Union of Microbiological Societies). Even within the same group of hepatitis viruses, for example, hepatitis B virus (HBV), which is a DNA virus, is eliminated by the immune mechanism in many cases except in the neonatal and infantile periods with immature immune potential, with some people experiencing the onset of acute hepatitis. By contrast, HCV often leads to persistent infection even in cases where an adult with mature immune mechanism is infected, because it avoids the host immune mechanism by a cause that remains unclear.

It is known that if chronic hepatitis is caused by persistent infection of HCV, then it progresses to liver cirrhosis and liver cancer at high probability, and that even if the cancer is extirpated by surgery, many patients experience recurrence of liver cancer due to inflammation that successively occurs at a non-cancer site. Also, a report is available that HCV infection is involved in dermal diseases such as chronic urticaria, lichen plunus, and cryoglobulinemic purpura (see Minami et al., Japanese Journal of Dermatology, vol.111(7), pp.1075-81, 2001).

In the envelop of HCV, E1 and E2, which are virus-derived structural proteins, are present, and these E1 and E2 proteins are thought to be mediator molecules on the HCV side in the pathway of the infection of HCV to cells, that is, from the adsorption of HCV to the cell surface to the entry into the cells (see K. Watashi et al., Cancer Science, vol. 94(11), pp.937-943, 2003).

Meanwhile, on the cell side, low density lipoprotein receptor (LDLR), scavenger receptor class B type I (SRBI), dendritic cell-specific intercellular adhesion molecule-3-grabbing nonintegrin (DC-SIGN; also referred to as CD209), liver or lymph node-specific intercellular adhesion molecule-3-grabbing nonintegrin (L-SIGN; also referred to as CD209L) and the tetraspanin CD81 have been reported to bind to envelop proteins of HCV respectively, and it has been suggested that these proteins may function as receptors on the cell side in the infection of HCV (see, for example, V. Racanelli et al., Trends in Immunology, vol.24(8), pp.456-464, 2003; P. Pileri et al., Science, vol.282, pp.938-941, 1998). However, little is known about the presence of receptor proteins other than those mentioned above and the mechanism for the cell infection of HCV (cell adsorption and entry into the cell).

Fibroblast growth factor receptor (FGFR) is said to be involved in a wide variety of biological phenomena, including early development and organogenesis, in cooperation with 23 kinds of fibroblast growth factor (FGF) ligands that have been found to date (see, for example, X. Coumoul et al., Birth Defects Research, (part C), vol.69, pp.286-304, 2003; B. Reuss et al., Cell and Tissue Research, vol.313, pp.139-157, 2003). Regarding findings concerning the functions of FGF and FGFR in the liver, there are many ones suggesting their involvement in the early developmental process; for example, it has been reported that FGF-1 and FGF-2 produced from the mesoderm in the differentiation from the foregut to hepatic precursor cells induced by co-culturing the cardiac mesoderm and foregut function as signal molecules for the differentiation induction, and that phosphorylated FGFR is present in the liver of the mouse early embryo during developing (see, for example, J. Jung et al., Science, vol.284, pp.1998-2003, 1999; GJ. Darlington, Current Opinion in Cell Biology, vol. 11, pp.678-682, 1999; SS. Sekhon et al., American Journal of Pathology, vol.164(6), pp.2229-2240, 2004). Also, a report is available that FGFR-4 is expressed in mature hepatocytes, and if this FGFR-4 is prevented from being expressed, liver dysfunctions represented by gall bladder depletion and the accumulation and increased secretion of bile acid, and the like occur (see, for example, X. Coumoul et al., Birth Defects Research, (part C), vol.69, pp.286-304, 2003). However, absolutely no report has been presented to date that FGFR is involved in the cell infection of HCV.

Currently, there is a demand for the establishment of effective therapeutic methods for hepatitis C; in particular, aside from symptomatic therapies to suppress inflammation with anti-inflammatory agents, there is a strong demand for the development of a drug that reduces HCV to the extent that does not cause inflammation, or eradicates HCV. Under these circumstances, drugs having an action to inhibit the infection of HCV to cells are expected to be potentially promising drugs because they are capable of suppressing the growth of HCV as a result of a repeated cycle of infection to host cells, replication, budding, and re-infection. However, because there has been no cell culture system for HCV to date, it has been the most important issue in HCV research to establish a reliable cell culture system.

To analyze the early process of the cell infection of HCV, the present inventors prepared a pseudotyped HCV coated with the envelope protein of HCV on the particle surface, and designed to express a reporter gene when infecting to cells, and constructed a highly sensitive cell infection assay system (see, Y. Matsuura et al., Virology, vol.286, pp. 263-275, 2001). Furthermore, the present inventors found using this assay system that HCV entered cells by endocytosis via the receptor protein of the cells, and that two envelope proteins of HCV are essential to the cell infection (ibidem).

### Disclosure of the Invention

Accordingly, the present invention is intended to provide a screening method and identification method useful for elucidating a novel membrane receptor protein necessary for the cell infection of HCV and the cell infection mechanism mediated by the protein and developing a pharmaceutical capable of inhibiting the cell infection of HCV by a novel action mechanism.

The present inventors diligently investigated the mechanism for the cell infection of HCV using a pseudotyped virus of HCV, found that (i) in cells having fibroblast growth factor receptor (FGFR)expressed forcedly therein, the infectivity of a pseudotyped virus of HCV increases, and that (ii) in cells having FGFR expressed forcedly therein, fibroblast growth factor (FGF) exhibits inhibitory action on the cell infection of the virus, and discovered that FGFR functioned as an HCV receptor in the initial infection process from the adsorption of HCV to cells to the entry into the cells.

Furthermore, the present inventors, while continuing a diligent investigation, obtained results suggesting that, among the members of the FGFR family, (i) FGFR-4 is involved mainly in the binding of HCV virus to surface protein, and (ii) FGFR-5 is involved mainly in the incorporation of HCV into cells.

From the findings above, the present inventors got an idea of utilizing FGFR as the target protein for screening or identification of compounds in order to develop a pharmaceutical having novel action mechanism capable of inhibiting the cell infection of HCV by inhibiting the interactions between FGFR and HCV, constructed an assay system for screening or identification by performing a selection or judgment of a compound having affinity for FGFR or a compound having the capability of blocking the binding of FGFR and HCV, using FGFR-expressing cells, a membrane fraction thereof or solubilized FGFR, and detection of intracellular incorporation of a pseudotyped virus of HCV in the presence of the compound, sequentially or simultaneously, and developed the present invention.

Accordingly, the present invention provides the following:
(A1) A screening method for a compound capable of inhibiting a cell infection of hepatitis C virus (HCV), which comprises a step of screening a test compound with the affinity for a fibroblast growth factor receptor (FGFR) as an index.
(A2) The method of (A1), wherein the aforementioned FGFR has a binding activity to a surface protein of HCV. (A3) The method of (A2), wherein the aforementioned FGFR is FGFR-4.
(A4) The method of (A2), wherein the aforementioned FGFR is FGFR-5.
(A5) The method of (A1), wherein the aforementioned FGFR has the capability of cell incorporation of HCV.
(A6) The method of (A5), wherein the aforementioned FGFR is FGFR-5.
(A7) The method of any one of (A1) to (A6), wherein a membrane fraction of a cell having an FGFR is used.
(A8) The method of any one of (A1) to (A6), wherein a cell having FGFR expressed forcedly therein.
(A9) The method of any one of (A1) to (A6), wherein a solubilized FGFR is used.
(A10) The method of any one of (A1) to (A6), which comprises the following steps:
   (a) a step of screening a test compound with an affinity for FGFR as an index; and
   (b) a step of evaluating the degree of cell infection of HCV in the presence of the compound having an affinity for FGFR screened in step (a).
(A11) The method of (A10), wherein the degree of cell infection of HCV is evaluated using a pseudotyped virus of HCV.
(A12) The method of any one of (A1) to (A6), wherein the aforementioned affinity is the binding activity between an FGF-binding site of FGFR and the test compound.
(A13) A screening kit for a compound capable of inhibiting cell infection of HCV, which comprises a lipid bilayer membrane containing FGFR and/or FGFR-expressing cell, and a pseudotyped virus of HCV.
(A14) An inhibitor of cell infection of HCV, which comprises a compound having the affinity for FGFR.
(A15) The inhibitor of (A14), wherein the aforementioned affinity is the binding activity between an FGF-binding site of FGFR and the compound.
(A16) An inhibitor of cell infection of HCV, which comprises a solubilized FGFR.
(A17) The inhibitor of (A16), wherein the aforementioned solubilized FGFR is a solubilized FGFR-4.
(A18) The inhibitor of (A16), wherein the aforementioned solubilized FGFR is a solubilized FGFR-5.

(1) A screening method for a compound inhibiting cell infection of hepatitis C virus (HCV), which comprises
   (a) a step of measuring an affinity of a test compound and fibroblast growth factor receptor (FGFR) having a binding activity to a surface protein of HCV and/or a capability of cell incorporation of HCV; and
   (b) a step of selecting the test compound having the affinity.
(2) The method of (1), further comprising
   (c) a step of measuring the degree of cell infection of HCV in the presence and absence of the selected test compound; and
   (d) a step of comparing both the measured values.
(3) The method of (2), wherein the degree of cell infection of HCV is measured using a pseudotyped virus of HCV.
(4) The method of any one of (1) to (3), wherein said FGFR is FGFR-4.
(5) The method of any one of (1) to (3), wherein said FGFR is FGFR-5.
(6) The method of any one of (1) to (5), wherein a membrane fraction of a cell having FGFR is used.
(7) The method of any one of (1) to (5), wherein a cell having FGFR expressed forcedly therein is used.
(8) The method of any one of (1) to (5), wherein a solubilized FGFR is used.
(9) The method of any one of (1) to (5), wherein said affinity is a binding activity between the test compound and an FGF-binding site of FGFR.
(10) A screening method for a compound inhibiting a cell infection of hepatitis C virus (HCV), which comprises
   (a) a step of measuring a capability of a test compound to block binding of HCV and fibroblast growth factor receptor (FGFR) having a binding activity to a surface protein of HCV; and
   (b) a step of selecting the test compound having the capability of blocking.
(11) The method of (10), further comprising
   (c) a step of measuring the degree of cell infection of HCV in the presence and absence of the selected test compound; and
   (d) a step of comparing both the measured values.
(12) The method of (11), wherein the degree of cell infection of HCV is measured using a pseudotyped virus of HCV.
(13) The method of any one of (10) to (12), wherein said FGFR is FGFR-4.
(14) The method of any one of (10) to (12), wherein said FGFR is FGFR-5.
(15) The method of any one of (10) to (14), wherein a membrane fraction of a cell having FGFR is used.
(16) The method of any one of (10) to (14), wherein a cell having FGFR expressed forcedly therein is used.
(17) The method of any one of (10) to (14), wherein a solubilized FGFR is used.
(18) The method of any one of (10) to (14), wherein said capability of blocking is a capability of the test compound to block the binding of HCV and an FGF-binding site of FGFR.
(19) A screening kit for a compound inhibiting cell infection of HCV, which comprises a lipid bilayer membrane containing FGFR and/or a FGFR-expressing cell, and a pseudotyped virus of HCV.
(20) A method of identifying a compound inhibiting cell infection of hepatitis C virus (HCV), which comprises
   (a) a step of measuring an affinity of a test compound and fibroblast growth factor receptor (FGFR) having a binding activity to a surface protein of HCV and/or a capability of cell incorporation of HCV; and.
   (b) a step of judging the test compound having the affinity.
(21) The method of claim 20, further comprising
   (c) a step of measuring the degree of cell infection of HCV in the presence and absence of the judged test compound; and
   (d) a step of comparing both the measured values.
(22) The method of (21), wherein the degree of cell infection of HCV is measured using a pseudotyped virus of HCV.
(23) The method of any one of (20) to (22), wherein said FGFR is FGFR-4.
(24) The method of any one of (20) to (22), wherein said FGFR is FGFR-5.
(25) The method of any one of (20) to (24), wherein a membrane fraction of a cell having FGFR is used.
(26) The method of any one of (20) to (24), wherein a cell having FGFR expressed forcedly therein is used.
(27) The method of any one of (20) to (24), wherein a solubilized FGFR is used.
(28) The method of any one of (20) to (24), wherein said affinity is a binding activity between the test compound and an FGF-binding site of FGFR.
(29) A method of identifying a compound inhibiting a cell infection of hepatitis C virus (HCV), which comprises
   (a) a step of measuring a capability of a test compound to block the binding of HCV and fibroblast growth factor receptor (FGFR) having a binding activity to a surface protein of HCV; and
   (b) a step of judging the test compound having the capability of blocking.
(30) The method of (29), further comprising
   (c) a step of measuring the degree of cell infection of HCV in the presence and absence of the judged test compound; and
   (d) a step of comparing both the measured values.
(31) The method of (30), wherein the degree of cell infection of HCV is measured using a pseudotyped virus of HCV.
(32) The method of any one of (29) to (31), wherein said FGFR is FGFR-4.
(33) The method of any one of (29) to (31), wherein said FGFR is FGFR-5.
(34) The method of any one of (29) to (33), wherein a membrane fraction of a cell having FGFR is used.
(35) The method of any one of (29) to (33), wherein a cell having FGFR expressed forcedly therein is used.
(36) The method of any one of (29) to (33), wherein a solubilized FGFR is used.
(37) The method of any one of (29) to (33), wherein said capability of blocking is a capability of the test compound to block the binding of HCV and an FGF-binding site of FGFR.
(38) A kit for identifying a compound inhibiting cell infection of HCV, which comprises a lipid bilayer membrane containing FGFR and/or a cell expressing FGFR, and a pseudotyped virus of HCV.
(39) An inhibitor of cell infection of HCV, which comprises a compound having an affinity for FGFR having a binding activity to a surface protein of HCV and/or a capability of cell incorporation of HCV.
(40) The inhibitor of (39), wherein said FGFR is FGFR-4.
(41) The inhibitor of (39), wherein said FGFR is FGFR-5.
(42) The inhibitor of any one of (39) to (41), wherein said compound is a low molecular compound.
(43) The inhibitor of any one of (39) to (41), wherein said compound is a ligand for FGFR.
(44) The inhibitor of (43), wherein said ligand for FGFR is FGF or a fragment thereof.
(45) The inhibitor of (43), wherein said ligand for FGFR is an antibody against FGFR-4.
(46) The inhibitor of (43), wherein said ligand for FGFR is an antibody against FGFR-5.
(47) The inhibitor of any one of (39) to (41), wherein said affinity is a binding activity between an FGF-binding site of FGFR and a compound.
(48) An inhibitor of cell infection of HCV, which comprises a solubilized FGFR based on FGFR having a binding activity to a surface protein of HCV and/or a capability of cell incorporation of HCV.
(49) The inhibitor of cell infection of HCV of (48), wherein said solubilized FGFR is a solubilized FGFR-4.
(50) The inhibitor of cell infection of HCV of (48), wherein said solubilized FGFR is a solubilized FGFR-5.
(51) An inhibitor of cell infection of HCV, comprising a compound having a capability of blocking the binding of HCV and FGFR having a binding activity to a surface protein of HCV.
(52) The inhibitor of (51), wherein said FGFR is FGFR-4.
(53) The inhibitor of (51), wherein said FGFR is FGFR-5.
(54) The inhibitor of any one of (51) to (53), wherein said compound is a low molecular compound.
(55) The inhibitor of any one of (51) to (53), wherein said compound is a ligand for FGFR.
(56) The inhibitor of (55), wherein said ligand for FGFR is FGF or a fragment thereof.
(57) The inhibitor of (55), wherein said ligand for FGFR is an antibody against FGFR-4.
(58) The inhibitor of (55), wherein said ligand for FGFR is an antibody against FGFR-5.
(59) The inhibitor of any one of (51) to (53), wherein said compound is a solubilized FGFR.
(60) The inhibitor of (59), wherein said solubilized FGFR is a solubilized FGFR-4.
(61) The inhibitor of (59), wherein said solubilized FGFR is a solubilized FGFR-5.
(62) The inhibitor of any one of (51) to (53), wherein said capability of blocking is a capability of the test compound to block the binding of HCV and an FGF-binding site of FGFR.
(63) A method of inhibiting cell infection of HCV, which comprises administering, to a patient in need of inhibition of cell infection of HCV, a therapeutically effective amount of a compound having an affinity for an FGFR having a binding activity to a surface protein of HCV and/or a capability of cell incorporation of HCV.
(64) A method of inhibiting cell infection of HCV, which comprises administering, to a patient in need of inhibition of cell infection of HCV, a therapeutically effective amount of a solubilized FGFR based on FGFR having a binding activity to a surface protein of HCV and/or a capability of cell incorporation of HCV.
(65) A method of inhibiting cell infection of HCV, which comprises administering, to a patient in need of inhibition of cell infection of HCV, a therapeutically effective amount of a compound having a capability of blocking the binding of HCV and FGFR having a binding activity to a surface protein of HCV.
(66) Use of a compound having an affinity for an FGFR having a binding activity to a surface protein of HCV and/or a capability of cell incorporation of HCV, for the manufacture of an inhibitor of cell infection of HCV.
(67) Use of a solubilized FGFR based on an FGFR having a binding activity to a surface protein of HCV and/or a capability of cell incorporation of HCV, for the manufacture of an inhibitor of cell infection of HCV.
(68) Use of a compound having a capability of blocking the binding of an FGFR having a binding activity to a surface protein of HCV and HCV, for the manufacture of an inhibitor of cell infection of HCV.

The screening method and identification method of the present invention are useful for developing a pharmaceutical having novel action mechanism capable of inhibiting the cell infection of HCV by inhibiting the interactions between fibroblast growth factor receptor (FGFR) and hepatitis C virus (HCV).

### Brief Description of the Drawings

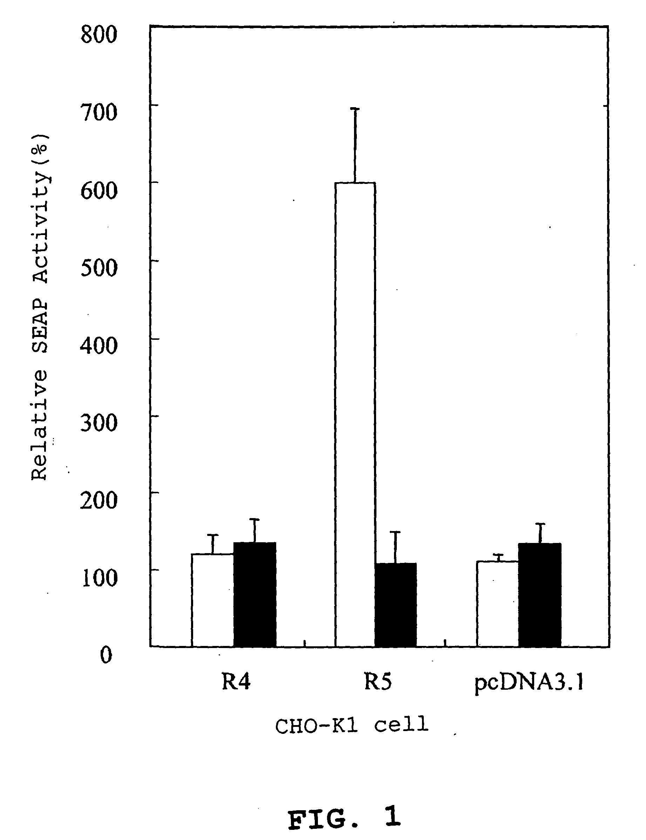

Fig. 1 shows the infectivity of ΔG/SEAP/HCV E1E2 to CHO-K1 cells having FGFR expressed forcedly therein. In the Figure, "R4" on the abscissa indicates CHO-K1 cells having FGFR-4 expressed forcedly therein, "R5" indicates CHO-K1 cells having FGFR-5 expressed forcedly therein, "pcDNA3.1" indicates CHO-K1 cells transfected with an empty pcDNA3.1 vector, and the ordinate indicates the relative SEAP activity (%) of each type of transfected cells to the relative value (Relative Light Units: RLU) of SEAP activity in the cell culture supernatant of native CHO-K1 cells as 100%. The □ bar shows the results of an inoculation of ΔG/SEAP/HCV E1E2, and the ■ bar shows the results of an inoculation of ΔG/SEAP/VSV G.

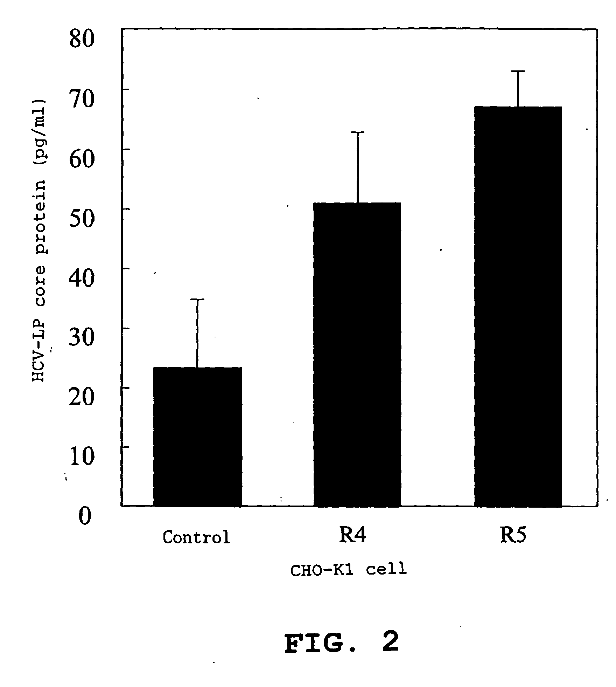

Fig. 2 shows the binding activity of HCV-LP to CHO-K1 cells having FGFR-4 expressed forcedly therein and CHO-K1 cells having FGFR-5 expressed forcedly therein. In the Figure, "Control" on the abscissa indicates native CHO-K1 cells, "R4" indicates CHO-K1 cells having FGFR-4 expressed forcedly therein, "R5" indicates CHO-K1 cells having FGFR-5 expressed forcedly therein, and the ordinate indicates the amount (pg/ml) of HCV-LP core protein adsorbed to cells.

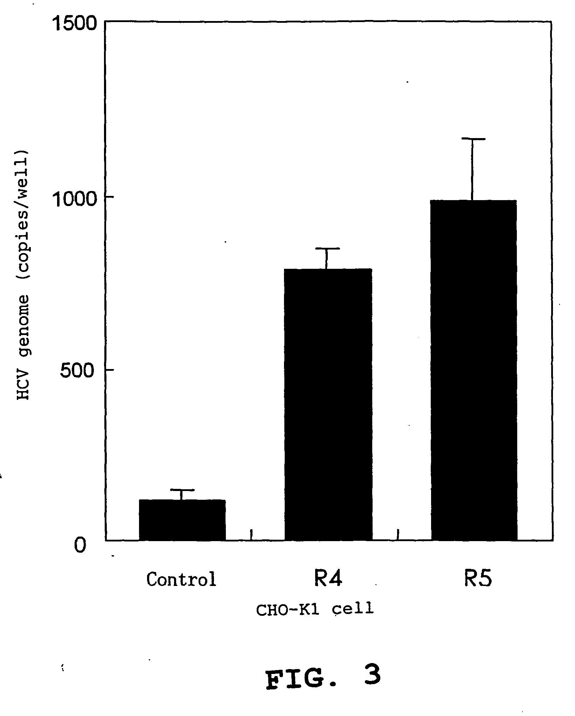

Fig. 3 shows the binding activity of HCV to CHO-K1 cells having FGFR expressed forcedly therein. In the Figure, "Control" on the abscissa indicates native CHO-K1 cells, "R4" indicates CHO-K1 cells having FGFR-4 expressed forcedly therein, "R5" indicates CHO-K1 cells having FGFR-5 expressed forcedly therein, and the ordinate indicates the copy number of HCV genome per well.

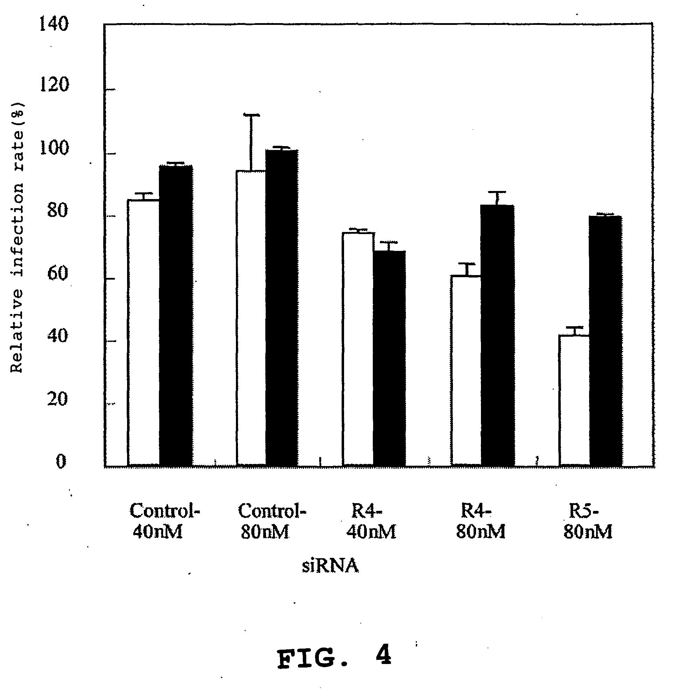

Fig. 4 shows the infectivity of ΔG/GFP/HCV E1E2 to Huh7 cells having FGFR knocked down with a siRNA. In the Figure, "Control-" on the abscissa indicates non-knockdown Huh7 cells using a siRNA designed not to knock down FGFR, "R4-" indicates FGFR-4 knockdown Huh7 cells, "R5-" indicates FGFR-5 knockdown Huh7 cells, and the concentrations indicate the amount of siRNA used for transfection. The ordinate indicates the relative infection rate (%) of each type of siRNA-treated cells to the infection level of non-siRNA-transfected Huh7 cells as 100%. The □ bar shows the results of an inoculation of ΔG/GFP/HCV E1E2, and the ■ bar shows the results of an inoculation of ΔG/GFP/VSV-G.

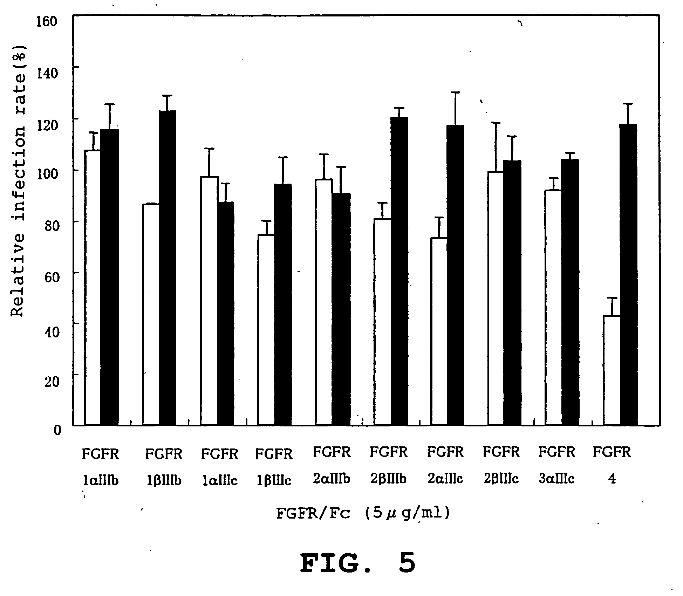

Fig. 5 shows the effects of various FGFR/Fc on the infectivity of ΔG/SEAP/HCV E1E2 to HepG2 cells. In the Figure, the abscissa indicates the FGFR/Fc used, and the ordinate indicates the relative infection rate (%) of each type of FGFR/Fc-treated cells to the infection level of cells inoculated with ΔG/SEAP/HCV E1E2 alone as 100%. The □ bar shows the results of an inoculation of ΔG/SEAP/HCV E1E2, and the ■ bar shows the results of an inoculation of ΔG/SEAP/VSV G.

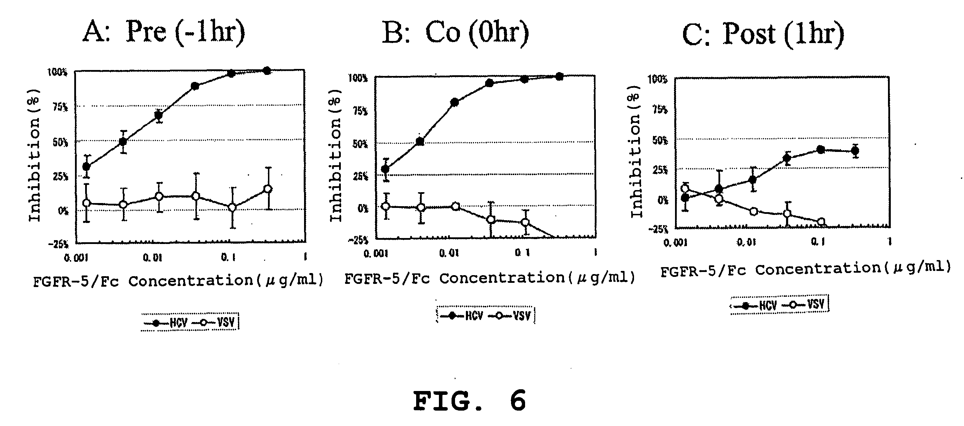

Fig. 6 shows the effects of FGFR-5/Fc on the infectivity of ΔG/SEAP/HCV E1E2 to HepG2 cells. A: Pre (-1 hr) shows the results obtained by mixing FGFR-5/Fc and the virus 1 hour before inoculation of the virus to the cells; B: Co (0 hr) shows the results obtained by inoculating the virus into the cells at the same time as mixing FGFR-5/Fc and the virus; and C: Post (1 hr) shows the results obtained by adding FGFR-5/Fc to the virus-inoculated cells 1 hour after inoculation of the virus to the cells. In each graph in the Figure, the abscissa indicates the concentration of FGFR-5/Fc. "-•-HCV" indicates the inhibition rate (%) of the infection of ΔG/SEAP/HCV E1E2 to the cells, and "-O-VSV" indicates the inhibition rate (%) of the infection of ΔG/SEAP/VSV G, which is the control virus, to the cells, each expressed as mean±S.D.

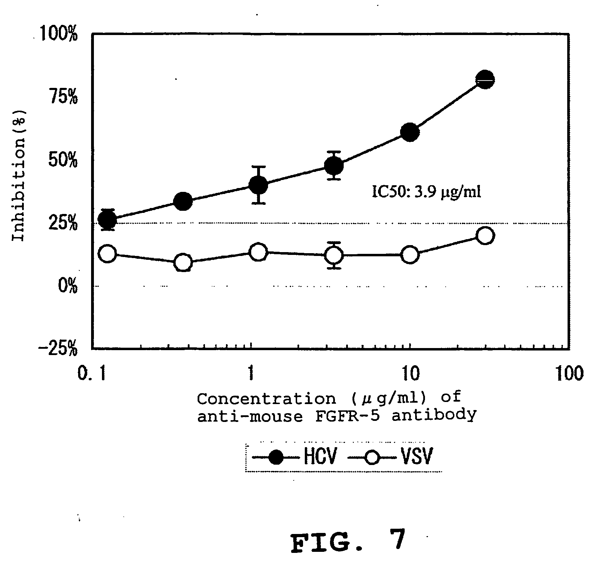

Fig. 7 shows the effects of anti-mouse FGFR-5 polyclonal antibody on the infectivity of ΔG/SEAP/HCV E1E2 to HepG2 cells. The abscissa of the graph indicates the concentration of anti-mouse FGFR-5 polyclonal antibody. "-•-HCV" indicates the inhibition rate (%) of the infection of ΔG/SEAP/HCV E1E2 to the cells, and "-O-VSV" indicates the inhibition rate (%) of the infection of ΔG/SEAP/VSV G, which is the control virus, to the cells, each expressed as mean±S.D.

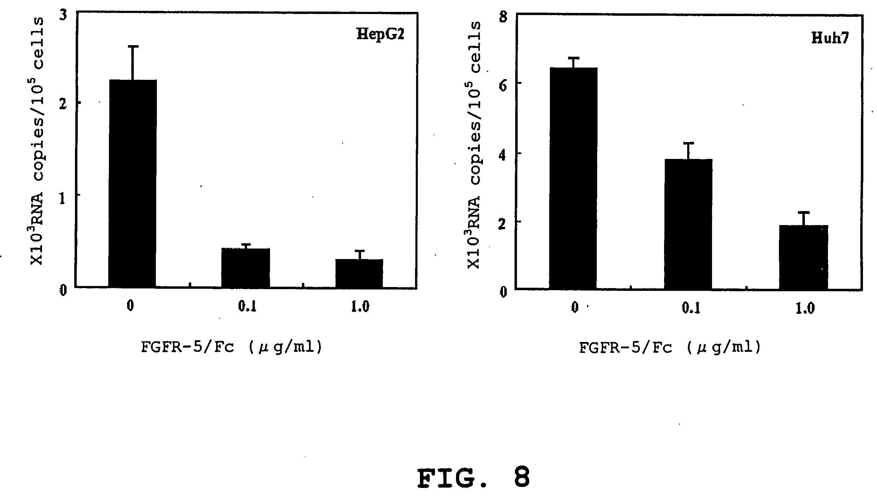

Fig. 8 shows the effects of solubilized FGFR-5 on the binding activity of HCV in patient plasma to cells. The left graph in the figure shows the results in HepG2 cells. The right graph in the figure shows the results in Huh7 cells. The abscissa indicates the concentration of solubilized FGFR-5 (FGFR-5/Fc). The ordinate indicates the RNA amount of the HCV bound to the cells.

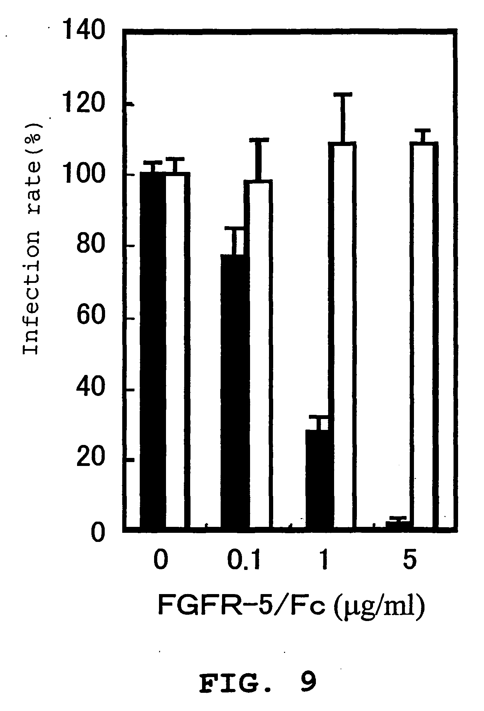

Fig. 9 shows the effects of solubilized FGFR-5 on the infectivity of a pseudotyped virus of HCV to human hepatocyte primary culture. The abscissa indicates the concentration of solubilized FGFR-5 (FGFR-5/Fc). The ordinate indicates the relative infection rate (%) to the infection level of cells inoculated with ΔG/SEAP/HCV E1E2 only as 100%. The ■ bar shows the results of an inoculation of ΔG/SEAP/HCV E1E2, and the □ bar shows the results of an inoculation of ΔG/SEAP/VSV G.

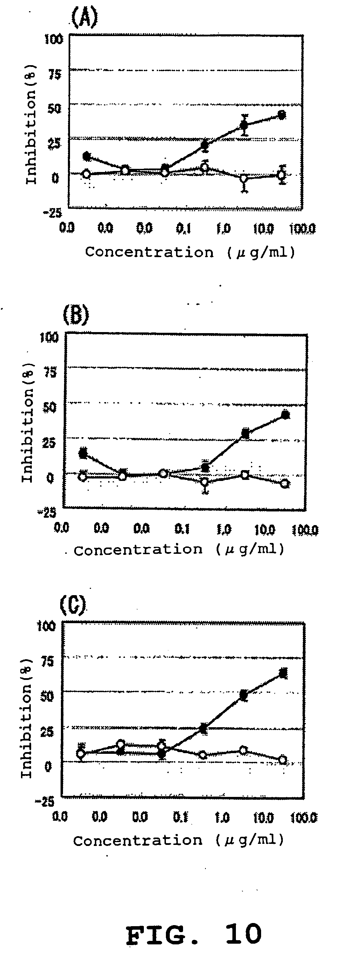

Fig. 10 shows the effects of anti-FGFR-5 monoclonal antibodies on the infectivity of HCV pseudotyped virus to HepG2 cells. The abscissa indicates the concentration of anti-FGFR-5 monoclonal antibody. The ordinate indicates the infection inhibition rate (%). "-•-" shows the results of an inoculation of ΔG/SEAP/HCV E1E2, and "-O-" shows the results of an inoculation of ΔG/SEAP/VSV G. (A) shows the results obtained with the anti-FGFR-5 monoclonal antibody R5C7-10. (B) shows the results obtained with the anti-FGFR-5 monoclonal antibody R5D14-19. (C) shows the results obtained with a mixture of R5C7-10 and R5D14-19.

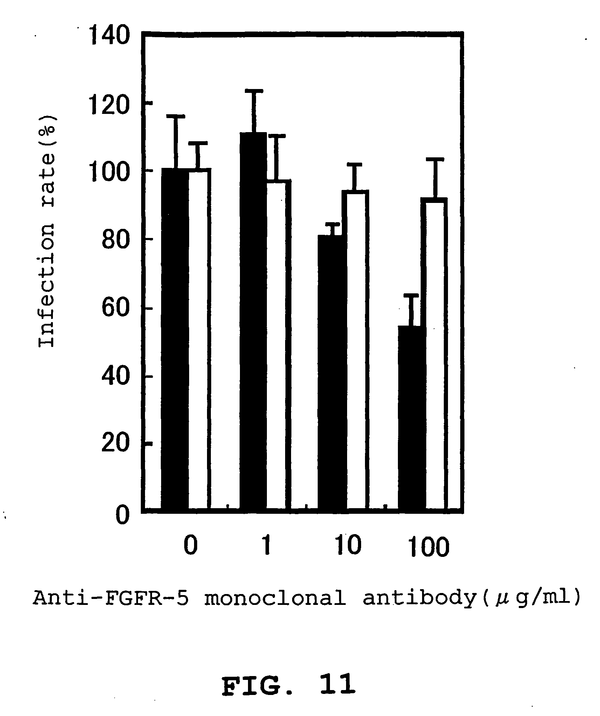

Fig. 11 shows the effects of anti-FGFR-5 monoclonal antibody on the infectivity of a pseudotyped virus of HCV to human hepatocyte primary culture. The abscissa indicates the concentration of anti-FGFR-5 monoclonal antibody. The ordinate indicates relative infection rate (%) to the infection level of cells inoculated with ΔG/SEAP/HCV E1E2 only as 100%. The ■ bar shows the results of an inoculation of ΔG/SEAP/HCV E1E2, and the □ bar shows the results of an inoculation of ΔG/SEAP/VSV G.

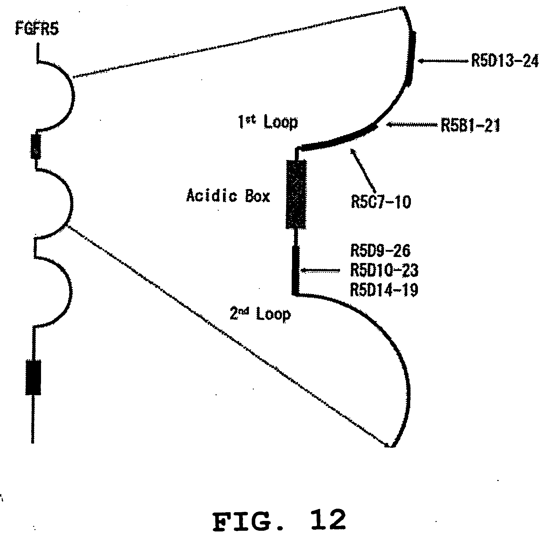

Fig. 12 is a scheme showing the epitopes of FGFR5 recognized respectively by various anti-FGFR-5 monoclonal antibodies prepared on the basis of Examples of the present invention.

### Best Mode for Embodying the Invention

The present invention relates to a screening method for a compound that inhibits the cell infection of hepatitis C virus (HCV), which comprises measuring the affinity for fibroblast growth factor receptor (FGFR) to select a test compound.

In another aspect, the present invention relates to a screening method for a compound that inhibits the cell infection of hepatitis C virus (HCV), which comprises measuring the capability of blocking the binding of fibroblast growth factor receptor (FGFR) and HCV to select a test compound.

In still another aspect, the present invention relates to an identification method for a compound that inhibits the cell infection of hepatitis C virus (HCV), which comprises using any of the above-described measurements to judge a test compound in accordance with the above-described screening method.

In the present invention, "screening" and "screen" denote an operation for sorting a compound having a particular property from a group consisting of a plurality of compounds. In this operation, the process for finally sorting such a compound on the basis of a result showing the presence and absence of the property obtained in a relatively early stage is referred to as "selection" or "select".

Meanwhile, "identification" and "identify" denote an operation for determining whether or not a certain compound or each of a plurality of compounds has a particular property. In this operation, the process for making a final judgment on the basis of a result showing the presence and absence of the property obtained in a relatively early stage is referred to as "judgment" or "judge". When these operations are performed on a plurality of compounds, these may be performed separately or concurrently. Therefore, "identification" and "identify" include the above-described "screening" and the above-described "screen", respectively.

"Fibroblast growth factor receptor" or "FGFR" as used herein means the family of transmembrane tyrosine kinases identified as a receptor that activates intracellular signal transduction with fibroblast growth factor (FGF) as the ligand. Members of this family have high mutual homology, and to date, four kinds, FGFR-1 to FGFR-4, have been known; more recently, FGFR-5 (FGFRL1) has been reported as a new member of the FGFR family, lacking the intracellular tyrosine kinase domain while having a high homology to these four kinds (see Biochemica et Biophysica Acta Vol.1518, pp.152-156, 2001; Genomics Vol.69, pp.275-279, 2000). These FGFRs, as shown herein, are mediator molecules that function as receptors on the cell side in the cell infection of HCV. Therefore, all receptors for FGF ligand, including those remaining unidentified, can be receptors of HCV. That is, "fibroblast growth factor receptor" or "FGFR" as used herein includes not only FGFR-1 to FGFR-5, which are already known as described above, but also unknown receptors for FGF ligand.

In the present invention, the FGFR used as the target protein in screening or identification means a human-derived FGFR or substantially the same protein thereas. Here, "substantially the same" refers to an amino acid sequence having a homology of about 60% or more, preferably about 70% or more, more preferably about 80% or more, particularly preferably about 90% or more, most preferably about 95% or more, to the amino acid sequence of human-derived FGFR, wherein the protein having the amino acid sequence has substantially the same quality of activity as human-derived FGFR. Here, "a homology" means the proportion (%) of identical amino acid residues and analogous amino acid residues to all overlapping amino acid residues in the optimal alignment where two amino acid sequences are aligned using a mathematical algorithm known in the technical field (preferably, the algorithm is such that a gap can be inserted into one or both of the sequences for an optimal alignment). "Analogous amino acid" means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (GIn, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such analogous amino acids is expected not to change the phenotype of proteins (i.e., conservative amino acid substitution). Specific examples of the conservative amino acid substitution are well known in the technical field and are described in various documents (see, for example, Bowie et al., Science, 247:1306-1310 (1990)).

Algorithms to determine amino acid sequence homology include, for example, but are not limited to, the algorithm described in Karlin et al., Proc. Natl. Acad. Sci. USA, 90:5873-5877 (1993) [the algorithm is incorporated in the NBLAST and XBLAST programs (version 2.0) (Altschul et al., Nucleic Acids Res., 25:3389-3402 (1997))], the algorithm described in Needleman et al., J. Mol. Biol., 48:444-453 (1970) [the algorithm is incorporated in the GAP program in the GCG software package], the algorithm described in Myers and Miller, CABIOS, 4:11-17 (1988) [the algorithm is incorporated in the ALIGN program (version 2.0), which is part of the CGC sequence alignment software package], the algorithm described in Pearson et al., Proc. Natl. Acad. Sci. USA, 85:2444-2448 (1988) [the algorithm is incorporated in the FASTA program in the GCG software package] and the like.

Although the type of FGFR is not subject to limitation, FGFR-4 or FGFR-5 is preferably used, and FGFR-5 is more preferably used. As suggested from an Example below, FGFR-4 particularly has "a binding activity to a surface protein of HCV", and can be involved mainly in cell adsorption in the cell infection of HCV. On the other hand, FGFR-5 has "a binding activity to a surface protein of HCV" and also has "the capability of cell incorporation of HCV", and can be involved in both cell adsorption and entry into the cell in the cell infection of HCV.

"A binding activity to a surface protein of HCV" means the capability of binding to "a surface protein of HCV". Here, "a surface protein of HCV" denotes a protein present in the envelop of HCV; as representative examples thereof, E1 and E2 proteins, which are HCV virus-derived structural proteins thought to be mediator molecules on the HCV side in the infection of HCV to cells, can be mentioned (see, K. Watashi et al., Cancer Science, vol.94(11), pp.937-943, 2003).

"The capability of cell incorporation of HCV" means the capability of fusion of HCV and cells, that is, the capability of incorporating HCV adhering to the cell surface into cells.

In FGFR-4 and FGFR-5, some splice variants are known, all of which may be used. Therefore, as the FGFR as used herein, preferably, a protein having an amino acid sequence having a homology of about 60% or more, preferably about 70% or more, more preferably about 80% or more, particularly preferably about 90% or more, most preferably about 95% or more, to the amino acid sequence shown by GenBank accession number:NP_002002 (that is, the amino acid sequence consisting of the full-length ORF of human FGFR-4) or the amino acid sequence shown by GenBank accession number:NP_068742 (that is, the amino acid sequence consisting of the full-length ORF of human FGFR-5), can be mentioned.

Examples of substantially the same quality include an activity to promote cell adsorption of HCV, an activity to promote entry into the cell of HCV and the like. "Substantially the same quality" means that the properties of the proteins are qualitatively (e.g., physiologically or pharmacologically) equivalent to each other. Accordingly, it is preferable that the proteins are equivalent to each other in terms of quantitative factors such as the extent of the above-described activity (e.g., about 0.01 to 100 times, preferably about 0.1 to 10 times, more preferably 0.5 to 2 times), but may be different.

As the method of measuring the above-described activity of FGFR, a method comprising measuring the incorporation activity of a pseudotyped virus of HCV (capable of expressing a reporter gene when infecting a cell, as described below) by FGFR-expressing cells with the expression of a reporter gene (for example, green fluorescent protein (GFP), alkaline phosphatase, luciferase, beta-galactosidase and the like) as the index, a method comprising quantifying HCV-LP (detailed description is given below) that binds to FGFR-expressing cells by, for example, an ELISA test using an antibody that recognizes a constituent component of HCV-LP, and the like, a method comprising measuring the degree of binding of FGFR (for example, intact FGFR-expressing cells, a membrane fraction of the cells, the solubilized FGFR described below and the like) and the above-described pseudotyped virus by a competitive binding test with labeled FGF, and the like can be mentioned, but are not to be construed as limiting.

Also, as substantially the same protein as human-derived FGFR used in the present invention, a protein comprising an amino acid sequence of human FGFR, for example, (1) an amino acid sequence resulting from the deletion of 1 or 2 or more (preferably about 1 to 30, preferably about 1 to 10, more preferably 1 to 5) amino acids from the amino acid sequence of human FGFR-4 shown by GenBank accession number:NP_002002 or the amino acid sequence of human FGFR-5 shown by GenBank accession number:NP_068742, (2) an amino acid sequence resulting from the addition of 1 or 2 or more (preferably about 1 to 30, preferably about 1 to 10, more preferably 1 to 5) amino acids to the amino acid sequence of human FGFR-4 shown by GenBank accession number:NP_002002 or the amino acid sequence of human FGFR-5 shown by GenBank accession number:NP_068742, (3) an amino acid sequence resulting from the insertion of 1 or 2 or more (preferably about 1 to 30, preferably about 1 to 10, more preferably 1 to 5) amino acids into the amino acid sequence of human FGFR-4 shown by GenBank accession number:NP_002002 or the amino acid sequence of human FGFR-5 shown by GenBank accession number:NP_068742, (4) an amino acid sequence resulting from the substitution of 1 or 2 or more (preferably about 1 to 30, preferably about 1 to 10, more preferably 1 to 5) amino acids in the amino acid sequence of human FGFR-4 shown by GenBank accession number:NP_002002 or the amino acid sequence of human FGFR-5 shown by GenBank accession number:NP_068742 with other amino acids, or (5) an amino acid sequence comprising a combination thereof, and having substantially the same quality of activity as human FGFR-4 or human FGFR-5, and the like are also included. Here, "substantially the same quality of activity" has the same definition as described above. Also, a measurement of "substantially the same quality of activity" can be performed in the same manner as described above.

When an amino acid sequence is deleted, added, inserted or substituted as described above, the position of the deletion, addition, insertion or substitution is not subject to limitation, as long as the protein retains its activity.

For the proteins specified by the amino acid sequence in the present specification, the left end is the N terminal (amino terminal) and the right end is the C terminal (carboxyl terminal) in accordance with the conventional peptide marking. Regarding the FGFR to be used in the present invention, including human FGFR-4 shown in GenBank registration No.: NP_002002 and human FGFR-5 shown in GenBank registration No.: NP_068742, the C terminal may be any of a carboxyl group (-COOH), a carboxylate (-COO⁻), an amide (-CONH₂), or an ester (-COOR).

Here, as R in the ester, a C₁₋₆ alkyl group such as methyl, ethyl, n-propyl, isopropyl, and n-butyl; a C₃₋₈ cycloalkyl group such as cyclopentyl and cyclohexyl; a C₆₋₁₂ aryl group such as phenyl and α -naphthyl; a phenyl-C₁₋₂ alkyl group such as benzyl and phenethyl; a C₇₋₁₄ aralkyl group such as an α -naphthyl-C₁₋₂ alkyl group such as α -naphthylmethyl; a pivaloyloxymethyl group; and the like are used.

When the protein to be used in the present invention has a carboxyl group (or a carboxylate) at a position other than the C terminal, a protein wherein the carboxyl group is amidated or esterified is also included in the protein of the present invention. In this case, as the ester, the above-described ester at the C terminal, and the like, for example, are used.

Furthermore, the protein to be used in the present invention also includes a protein wherein the amino group of the N terminal amino acid residue (e.g., methionine residue) is protected by a protecting group (for example, C₁₋₆ acyl groups such as C₁₋₆ alkanoyl groups such as formyl group and acetyl group, and the like), a protein wherein the N terminal glutamine residue, which is produced upon cleavage in vivo, has been converted to pyroglutamic acid, a protein wherein a substituent (for example, -OH, -SH, amino group, imidazole group, indole group, guanidino group, and the like) on a side chain of an amino acid in the molecule is protected by an appropriate protecting group (for example, C₁₋₆ acyl groups such as C₁₋₆ alkanoyl groups such as formyl group and acetyl group, and the like), a conjugated protein such as what is called a glycoprotein having a sugar chain bound thereto, and the like.

FGFR is a single transmembrane protein having a tyrosine kinase domain in the intracellular region on the C-terminal side thereof (but FGFR-5 lacks such a tyrosine kinase domain), and it is thought that in the binding to, or interactions with, extracellular factors, mainly the extracellular region on the N-terminal side plays an important role. Therefore, the FGFR used for measuring the affinity of a test compound for FGFR does not always need to be a full-length protein, and may be a partial peptide fragment thereof.

The partial peptide of FGFR used in the present invention may be any one, as long as it is a peptide having the same or substantially the same amino acid sequence as a partial amino acid sequence of an amino acid sequence of human-derived FGFR, for example, the amino acid sequence of human FGFR-4 shown by GenBank accession number:NP_002002 or the amino acid sequence of human FGFR-5 shown by GenBank accession number:NP_068742, and having substantially the same quality of activity as the aforementioned FGFR used in the present invention. Here, "substantially the same quality of activity" has the same definition as described above. Also, a measurement of "substantially the same quality of activity" can be performed in the same manner as described above.

Specifically, as the partial peptide, a peptide having at least 200 or more, preferably 300 or more, more preferably 400 or more amino acids of the constituent amino acid sequence of the FGFR used in the present invention, and the like are used. Also, the partial peptide is desirably a portion comprising at least all or part of the extracellular region of FGFR, for example, the region involved in the binding to FGF.

Also, the partial peptide of FGFR used in the present invention may have (1) 1 or 2 or more (preferably about 1 to 10, more preferably 1 to 5) amino acids deleted from the amino acid sequence thereof, or (2) 1 or 2 or more (preferably about 1 to 20, more preferably about 1 to 10, still more preferably 1 to 5) amino acids added to the amino acid sequence thereof, or (3) 1 or 2 or more (preferably about 1 to 20, more preferably about 1 to 10, still more preferably 1 to 5) amino acids inserted into the amino acid sequence thereof, or (4) 1 or 2 or more (preferably about 1 to 10, more preferably 1 to 5) amino acids substituted in the amino acid sequence thereof by other amino acids, or may comprise (5) a combination thereof.

For the partial peptide of FGFR of the present invention, the C terminal may be any of a carboxyl group (-COOH), a carboxylate (-COO⁻), an amide (-CONH₂), or an ester (-COOR). Here, as R in the ester, the same as those mentioned for FGFR above can be mentioned. When the partial peptide has a carboxyl group (or a carboxylate) at a position other than the C terminal, a partial peptide wherein the carboxyl group is amidated or esterified is also included in the partial peptide of FGFR of the present invention. In this case, as the ester, the above-described ester at the C terminal, and the like, for example, are exemplified. Furthermore, the partial peptide also includes a partial peptide wherein the amino group of the N terminal amino acid residue (e.g., methionine residue) is protected by a protecting group, a partial peptide wherein glutamine residue produced upon cleavage at the N terminal in vivo, has been converted to pyroglutamic acid, a partial peptide wherein a substituent on a side chain of an amino acid in the molecule is protected by an appropriate protecting group, a conjugated peptide such as what is called a glycopeptide having a sugar chain bound thereto, and the like as in the case of FGFR.

Furthermore, the FGFR used to measure the affinity of a test compound for FGFR or the capability of a test compound of blocking the binding of FGFR and HCV may be a fusion protein prepared by adding another protein or a partial peptide thereof to the C-terminus of the above-described "human-derived FGFR or substantially the same protein thereas" or "partial peptide of FGFR" via an optionally chosen peptide linker as required.

As examples of the fusion protein, what is called "solubilized FGFR", which has "another soluble protein or a partial peptide thereof" as "another protein or a partial peptide thereof" for the purpose of solubilizing FGFR, which is essentially a transmembrane protein, can be mentioned.

As "solubilized FGFR", a portion comprising at least all or part of the extracellular region of the N-terminal side of FGFR, for example, the region involved in the binding to FGF, is preferably utilized as "a partial peptide of FGFR". Also, as examples of "another soluble protein or a partial peptide thereof", the Fc portion of the immunoglobulin H chain can be mentioned. (Note that "solubilized FGFR" comprising at least all or part of the extracellular region of the N-terminal side of FGFR as "a partial peptide of FGFR", and utilizing the Fc portion of the immunoglobulin H chain as "another soluble protein or a partial peptide thereof" is generally referred to as "soluble FGFR".)

Such "solubilized FGFR" can of course be used to measure the affinity of FGFR and a test compound or the capability of a test compound of blocking the binding of FGFR and HCV, and is also expected as a pharmaceutical itself capable of inhibiting the cell infection of HCV because the solubilized FGFR itself potentially inhibits the interactions of HCV surface protein with FGFR on the cell surface by binding to the HCV surface protein.

As the salt of FGFR or partial peptide of FGFR or fused protein thereof to be used in the present invention, salts with physiologically acceptable acid (e.g., inorganic acid, organic acid) or base (e.g., alkali metal salt) and the like are used, and physiologically acceptable acid addition salts are particularly preferable. As such salts, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like used.

FGFR or a salt thereof to be used in the present invention can be prepared from human cells [e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or a corresponding precursor cell, stem cell or cancer cell thereof, and the like] or any tissue or organ where such cells are present [e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submaxillary gland, peripheral blood, prostate, testicle, ovary, placenta, uterus, bone, joint, adipose tissues (e.g.: brown adipose tissue, white adipose tissue), skeletal muscle, and the like] by a known protein purification method. Specifically, they can be isolated and purified by homogenizing a tissue or cell of the animal, extracting the homogenate with an acid and the like, and subjecting the extract to a combination of chromatographys such as reversed-phase chromatography, ion exchange chromatography and the like.

FGFR or a partial peptide of FGFR, or a fusion protein, or a salt thereof to be used in the present invention (hereinafter sometimes to be simply abbreviated as "FGFR") can also be produced according to a known peptide synthesis method.

The method of peptide synthesis may, for example, be any of solid phase synthesis or liquid phase synthesis. The desired protein can be produced by condensing a partial peptide or amino acids that can constitute the protein of the present invention and the remaining portion, and removing a protecting group when the product has the protecting group. Condensation and removal of protecting group can be achieved by known methods, for example, the methods described in (1)-(5) below.
(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima and Shunpei Sakakibara: Seikagaku Jikken Koza (Biochemical Experiment) 1, Tanpakushitsu no Kagaku (Chemistry of Proteins) IV, 205 (1977)
(5) Haruaki Yajima, ed.: Zoku Iyakuhin no Kaihatsu (A sequel to Development of Pharmaceuticals), Vol. 14, Peptide Synthesis, published by Hirokawa Shoten.

For the synthesis of FGFR of the present invention, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenylhydroxymethyl)phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired protein or the like according to various methods of condensation known per se. At the end of the reaction, the protein or a partial peptide is cleaved from the resin and at the same time various protecting groups are removed, and a reaction to form an intramolecular disulfide bond is carried out in a highly diluted solution to obtain the desired protein or a partial peptide or an amide thereof.

For the above-described condensation of protected amino acids, various activation reagents for protein synthesis may be used, with preference given to carbodiimides. As the carbodiimides, DCC, N,N'-diisopropylcarbodiimide, N-ethyl-N'-(3-dimethylaminopropyl)carbodiimide and the like can be used. For activation with these reagents, protected amino acids may be added directly to the resin along with a racemization inhibitor (for example, HOBt, HOOBt etc.), or may be added to the resin after being previously activated to the form of a symmetric acid anhydride, HOBt ester, or HOOBt ester.

Solvents to be used in the activation of protected amino acids or condensation with the resin can be selected as appropriate from among the solvents known to be usable for protein condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethylsulfoxide; pyridine; ethers such as dioxane and tetrahydrofuran; nitriles such as acetonitrile and propionitrile; esters such as methyl acetate and ethyl acetate; appropriate mixtures of these solvents, and the like can be used. Reaction temperature is selected as appropriate from the range known to be useful for protein bond formation reactions, and is normally selected as appropriate from the range of about -20°C to about 50°C. The activated amino acid derivative is normally used in an excess of about 1.5 to about 4 times. If a test using the ninhydrin reaction reveals insufficient condensation, the condensation can be completed by repeating the condensation reaction without splitting off the protecting groups. If the condensation is yet insufficient even after repeating the reaction, unreacted amino acids can be acetylated with acetic anhydride or acetylimidazole so that its influence on the subsequent reactions can be avoided.

Protection and protecting groups for the functional groups that should not be involved in the reaction of the raw material, splitting off the protecting groups, activation of the functional groups involved in the reaction, and the like can be selected as appropriate from among known groups or known means.

Examples of the protecting groups for the amino groups of the raw material include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like.

The carboxyl group can be protected, for example, by alkylesterification (e.g., esterification of linear, branched or cyclic alkyl such as methyl, ethyl, propyl, butyl, t-butyl, cyclopentyl, cyclohexyl, cycloheptyl, cyclooctyl, 2-adamantyl and the like), aralkylesterification (e.g., benzylester, 4-nitrobenzylester, 4-methoxybenzylester, 4-chlorobenzylester, benzhydrylesterification), phenacylesterification, benzyloxycarbonylhydrazidation, t-butoxycarbonylhydrazidation, tritylhydrazidation and the like.

The hydroxyl group of serine can be protected by, for example, esterification or etherification. Examples of groups suitable for this esterification include lower (C₁₋₆) alkanoyl groups such as acetyl group, aroyl groups such as benzoyl group, groups derived from carbonic acid, such as benzyloxycarbonyl group and ethoxycarbonyl group, and the like. As examples of groups suitable for the etherification, benzyl group, tetrahydropyranyl group, t-butyl group and the like can be mentioned.

Examples of the protecting group for the phenolic hydroxyl group of tyrosine include Bzl, Cl₂-Bzl, 2-nitrobenzyl, Br-Z, t-butyl and the like.

Examples of the protecting group for the imidazole of histidine include Tos, 4-methoxy-2,3,6-trimethylbenzenesulfonyl, DNP, benzyloxymethyl, Bum, Boc, Trt, Fmoc and the like.

As examples of the method used to remove (split off) the protecting group, catalytic reduction in a hydrogen gas stream in the presence of a catalyst such as Pd-black or Pd-carbon; acid treatment with anhydrous hydrogen fluoride, methanesulfonic acid, trifluoromethanesulfonic acid or trifluoroacetic acid, or a mixed solution thereof; treatment with a base such as diisopropylethylamine, triethylamine, piperidine or piperazine; reduction with sodium in liquid ammonia, and the like can be mentioned. The reaction of splitting off the protecting group by the above-described acid treatment is normally performed at a temperature of about -20°C to 40°C; in the acid treatment, addition of a cation scavenger such as anisole, phenol, thioanisole, meta-cresol, para-cresol, dimethylsulfide, 1,4-butanedithiol or 1,2-ethanedithiol is effective. The 2,4-dinitrophenyl group used as the protecting group for the imidazole of histidine is removed by thiophenol treatment; the formyl group used as the protecting group for the indole of tryptophan is removed by alkali treatment with dilute sodium hydroxide solution, dilute ammonia or the like, as well as by the above-described acid treatment in the presence of 1,2-ethanedithiol, 1,4-butanedithiol or the like.

As examples of those obtained by activation of the carboxyl group in the raw material, a corresponding acid anhydride, an azide, an activated ester [an ester with an alcohol (for example, pentachlorophenol, 2,4,5-trichlorophenol, 2,4-dinitrophenol, cyanomethyl alcohol, paranitrophenol, HONB, N-hydroxysuccimide, N-hydroxyphthalimide, or HOBt)] and the like are used. As examples of those obtained by activation of the amino group in the raw material, a corresponding phosphoric amide is used.

In another method of preparing an amide of the protein or partial peptide, for example, the α-carboxyl group of the carboxy terminal amino acid is first amidated and hence protected, and a peptide (protein) chain is elongated to a desired chain length toward the amino group side, thereafter the both proteins or partial peptides having the protecting group for the N terminal α -amino group of the peptide chain only removed and the protein or partial peptide having the protecting group for the C terminal carboxyl group only removed are prepared, and the protein or peptide are condensed in a mixed solvent described above. For details about the condensation reaction, the same as above applies. After the protected protein or peptide obtained by the condensation is purified, all protecting groups can be removed by the above-described method to yield a desired crude protein or peptide. By purifying this crude protein or peptide using various known means of purification, and freeze-drying the main fraction, a desired amide of the protein or peptide can be prepared.

In order to obtain esters of the protein or peptide, a desired ester of the protein or peptide can be prepared by, for example, condensing the α -carboxyl group of the carboxy terminal amino acid with a desired alcohol to yield an amino acid ester, and then treating the ester in the same manner as in the case of an amide of the protein or peptide.

The partial peptide of FGFR or a salt thereof of the present invention can also be produced by cleaving FGFR or a salt thereof obtained by any of the methods described above or below, with an appropriate peptidase.

The thus-obtained FGFR can be purified and isolated by a known purification method. Here, as the purification method, for example, solvent extraction, distillation, column chromatography, liquid chromatography, recrystallization, a combination of these and the like can be mentioned.

When the protein or partial peptide obtained by the above-mentioned method is in a free form, it can be converted to a suitable salt by a known method or a method analogous thereto; conversely, when the peptide is obtained in the form of a salt, the salt can be converted to a free form or other salt by a known method or a method analogous thereto.

FGFR can also be acquired by transfecting an expression vector comprising a nucleic acid, preferably a DNA, that encodes FGFR, into a host cell according to a known method, to cause the forced expression of FGFR.

As the DNA that encodes FGFR or a partial peptide thereof, a genomic DNA, a cDNA derived from the aforementioned human cells or tissue/organs, a synthetic DNA and the like can be mentioned. A genomic DNA and cDNA that encode FGFR or a partial peptide thereof can also be directly amplified by Polymerase Chain Reaction (hereinafter abbreviated as "the PCR method") and Reverse Transcriptase-PCR (hereinafter abbreviated as "the RT-PCR method") using a genomic DNA fraction and total RNA or mRNA fraction, prepared from the above-described cells/tissue, respectively as the template. Alternatively, a genomic DNA and cDNA that encode FGFR or a partial peptide thereof can also be respectively cloned from a genomic DNA library and cDNA library, prepared by inserting a fragment of genomic DNA, total RNA or mRNA prepared from the above-described cells/tissue into an appropriate vector, by the colony or plaque hybridization method or the PCR method and the like. The vector used for the library may be any of a bacteriophage, a plasmid, a cosmid, a phagemid or the like.

As examples of the DNA that encodes FGFR, a DNA comprising the base sequence of the entire coding region of human FGFR-4 shown by GenBank accession number:NM_002011 or the base sequence of the entire coding region of human FGFR-5 shown by GenBank accession number:NM_021923, or a DNA comprising a base sequence that hybridizes with the DNA under high-stringent conditions, and encoding a protein, partial peptide or fusion protein having substantially the same quality of activity (for example, activity to promote the cell adsorption of HCV, activity to promote the entry into the cell of HCV and the like) as the aforementioned protein comprising the amino acid sequence of FGFR-4 shown by GenBank accession number:NP_002002 or the amino acid sequence of FGFR-5 shown by GenBank accession number:NP_068742, and the like can be mentioned.

As examples of the DNA capable of hybridizing with the base sequence shown by GenBank accession number:NM_002011 or GenBank accession number:NM_021923 under high-stringent conditions, a DNA comprising a base sequence having a homology of about 60% or more, preferably about 70% or more, more preferably about 80% or more, particularly preferably about 90% or more, to the base sequence shown by GenBank accession number:NM_002011 or GenBank accession number:NM_021923, and the like are used.

Base sequence homology in the present description can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3). As preferable examples of other algorithms for determining base sequence homology, the above-described amino acid sequence homology calculation algorithm can also be mentioned.

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under high-stringent conditions.

High-stringent conditions include, for example, conditions involving a sodium salt concentration of about 19 to 40mM, preferably about 19 to 20mM, and a temperature of about 50 to 70°C, preferably about 60 to 65°C. In particular, a case wherein the sodium salt concentration is about 19 mM and the temperature is about 65°C is preferred. Those of ordinary skill in the art can easily adjust a desired stringency by appropriately changing salt concentration of a hybridization solution, temperature of hybridization reaction, probe concentration, probe length, number of mismatch, time of hybridization reaction, salt concentration of washing, temperature of washing and the like.

The cloned DNA can be used as is, or after digestion with a restriction enzyme or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

An expression vector comprising DNA that encodes the FGFR or partial peptide thereof can be produced by, for example, cutting out a desired DNA fragment from the DNA that encodes the FGFR and ligating the DNA fragment downstream of a promoter in an appropriate expression vector.

As the expression vector, animal viruses such as retrovirus, vaccinia virus and the like; pA1-11, pXT1, pRc/CMV, pRc/RSV, pcDNAI/Neo, pME18S and the like can be used.

The promoter may be any promoter, as long as it is appropriate for the host used to express the gene. For example, the SRα promoter, the SV40 promoter, the LTR promoter, the CMV (cytomegalovirus) promoter, the HSV-TK promoter and the like are used.

Useful expression vectors include, in addition to the above, those optionally harboring an enhancer, a splicing signal, a polyA addition signal, a selection marker, an SV40 replication origin (hereinafter also abbreviated as SV40ori) and the like. As examples of the selection marker, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance) and the like can be mentioned. In particular, when a Chinese hamster cell lacking the dhfr gene is used in combination with the dhfr gene as the selection marker, a target gene can also be selected using a thymidine-free medium.

Gene transfection into a host cell can be performed, for example, according to a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973).

As the medium used for cell culture, for example, MEM medium [Science, vol. 122, 501 (1952)], DMEM medium [Virology, vol. 8, 396 (1959)], RPMI 1640 medium [The Journal of the American Medical Association, vol. 199, 519 (1967)], 199 medium [Proceeding of the Society for the Biological Medicine, vol. 73, 1 (1950)] and the like, which contain about 5 - 20% of fetal bovine serum (FBS), are used. The medium's pH is preferably about 6 to 8. Cultivation is normally carried out at about 30°C to about 40°C for about 15 to about 60 hours. As necessary, the culture may be aerated or agitated.

FGFR may be provided in any form, as long as its "affinity" for the test compound or the test compound's its "capability of blocking" its "binding to HCV" can be measured.

"Affinity" means the binding activity of FGFR and a test compound (that is, the capability of binding specifically); although the binding site is not subject to limitation, the compound having the affinity preferably has a binding activity to the extracellular region of FGFR, more preferably has a binding activity to the FGF-binding site of FGFR. As a result of such affinity (binding activity), the compound acts on FGFR antagonistically to HCV, and is capable of inhibiting the cell adsorption/entry into the cell of HCV mediated by FGFR.

"Binding to HCV" means the binding of FGFR and HCV, and is represented by the binding of both two proteins resulting from the exhibition of the above-described "binding activity to a surface protein of HCV" by FGFR. Therefore, the test compound's "capability of blocking" the "binding to HCV" means the capability of the test compound of inhibiting this binding, and the mode of inhibition thereof is not subject to limitation; for example, because a compound having the above-described "affinity" for FGFR acts antagonistically to HCV to inhibit the binding, it consequently becomes a compound having "the capability of blocking". As another example of the compound having "the capability of blocking", a compound that inhibits the binding of FGFR and HCV by acting on HCV antagonistically to FGFR can be mentioned. A preferable example of such a compound is the above-described "solubilized FGFR".

For example, in the screening method and identification method of the present invention, FGFR can be provided in the form of natively FGFR-expressing cells (for example, HepG2, Huh7, FLC4, HEK293, A549 and the like), cells having FGFR expressed forcedly therein by transfecting an expression vector comprising a nucleic acid, preferably a DNA, that encodes FGFR (for example, in addition to the above-described cells, those obtained from FGFR non-expressing cells such as HeLa cells and CHO cells as the host cells are also included), a membrane fraction of any of the above-described cells, a proteoliposome wherein isolated FGFR is embedded in an artificial lipid bilayer membrane, the FGFR protein per se completely or incompletely purified from the above-described cells or a tissue/organ from which they are derived, a fusion protein of FGFR, which includes solubilized FGFR, and the like, but these forms are not to be construed as limiting.

When FGFR-expressing cells are used to evaluate the affinity for FGFR, the degree of the cell infection of HCV can be evaluated after confirmation of the affinity or at the same time as the evaluation of the affinity. Just in the same manner, when FGFR-expressing cells are used to evaluate the capability of blocking the binding to HCV, the degree of the cell infection of HCV can be evaluated after confirmation of the capability of blocking or at the same time as the evaluation of the capability of blocking. Because some of natively FGFR-expressing cells can be of low expression levels, in one preferred mode of embodiment, the present invention can use cells having any type of FGFR expressed forcedly therein as the source of FGFR.

When cells having FGFR are used in the present invention, once a compound having the affinity for FGFR or the capability of blocking the binding of FGFR and HCV is discovered by a screening method or identification method, it is possible to subsequently add, to the system, HCV or a pseudotyped virus that mimics the initial infection process of HCV to evaluate the inhibitory effect of this compound on the infectivity of HCV to cells.

In another mode of embodiment, the present invention can use a membrane fraction of cells having any type of FGFR as the source of FGFR.

When a membrane fraction of cells having FGFR is used, screening can be performed more conveniently than using cells having FGFR; therefore, this mode of embodiment can be useful in primary (preliminary) screening for a large amount of test compound and the like. The membrane fraction can be prepared by, for example, homogenizing cells having FGFR in an appropriate buffer solution in the presence of various proteinase inhibitors, or suspending the cells using a cell disruptor such as Polytron, or destroying the cells by low osmotic pressure shock, or destroying the cell membrane by sonication, and then performing a density gradient centrifugation method using various media (for example, can be prepared by centrifuging the cell homogenate at about 1,000 x g, recovering the supernatant, and then performing density gradient centrifugation at about 100,000 x g and recovering the sediment, and the like).

In still another mode of embodiment of the present invention, as the FGFR, a purified FGFR protein regenerated in an artificial lipid bilayer membrane can be used. The FGFR can be purified from, for example, the above-described membrane fraction of cells having FGFR, by affinity chromatography using the anti-FGFR antibody described below, and the like. Alternatively, the FGFR can also be purified from the above-described recombinant cells incorporating an expression vector comprising a DNA that encodes the FGFR, by affinity chromatography and the like using an anti-FGFR antibody, His-tag, GST-tag and the like.

As the lipid that constitutes an artificial lipid bilayer membrane, phosphatidyl choline (PC), phosphatidyl serine (PS), cholesterol (Ch), phosphatidyl inositol (PI), phosphatidyl ethanolamine (PE) and the like can be mentioned, and a mixture of 1 kind or 2 kinds or more thereof in an appropriate ratio is preferably used.

For example, an artificial lipid bilayer membrane (proteoliposome) incorporating FGFR can be prepared by the method described below. Specifically, first, an appropriate amount of a chloroform solution of a mixed lipid of PC:PI:Ch=12:12:1 is taken in a glass tube, and the chloroform is evaporated with gaseous nitrogen vapor to dry the lipid in a film-like form, after which an appropriate buffer solution is added to suspend the lipid, which is then uniformly dispersed by sonication, and a buffer solution containing a surfactant such as sodium cholate is further added to completely suspend the lipid. An appropriate amount of purified FGFR is added thereto, and the suspension is incubated while being occasionally stirred in ice for about 20 to 30 minutes, after which it is dialyzed against an appropriate buffer solution. By centrifuging at about 100,000 x g for 30 to 60 minutes, and recovering the sediment, the desired proteoliposome can be obtained.

In still another mode of embodiment of the present invention, solubilized FGFR can be used as the FGFR.

When solubilized FGFR is used, screening can be performed more conveniently than using a membrane fraction of cells having FGFR or proteoliposome. Solubilized FGFR can be acquired from, for example, recombinant cells incorporating an expression vector comprising a DNA that encodes the same according to a known method of soluble protein purification, and additionally, a commercial product (for example, FGFR/Fc chimera commercially available from Sigma and the like) can be used.

The "test compound" used in the present invention may be any known compound or novel compound; examples thereof include, but are not limited to, nucleic acids, saccharides, lipids, proteins, peptides, antibodies, organic or inorganic low molecular compounds, organic or inorganic high molecular compounds, compound libraries prepared using combinatorial chemistry technology, random peptide libraries prepared by the solid phase synthesis or phage display method, or natural ingredients derived from microorganisms, animals, plants and the like, and the like.

Such test compound includes a compound already known to act as an antagonist of FGFR. Such compound includes, for example, the following: Troponin 1,

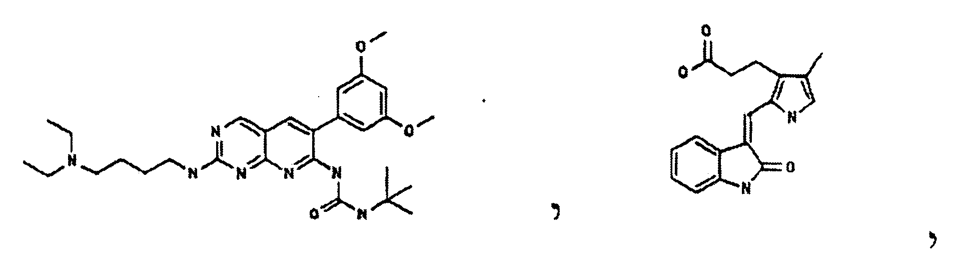

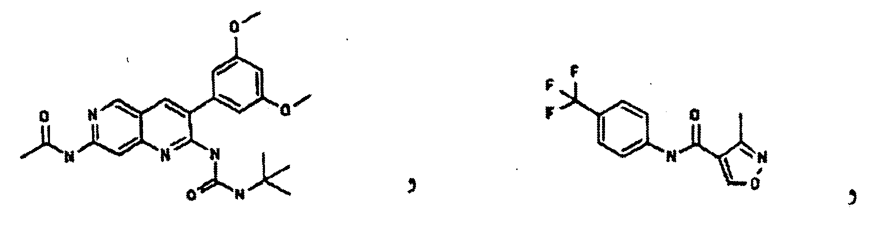

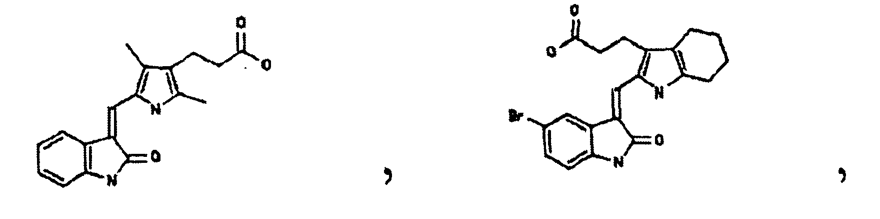

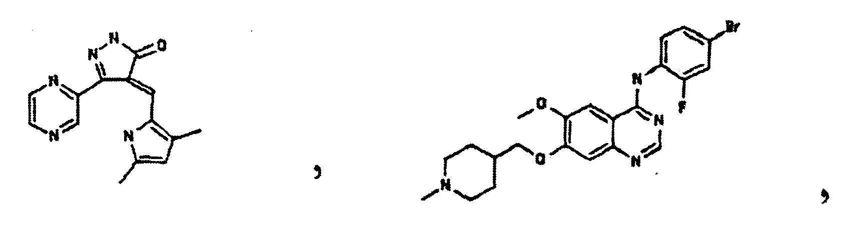

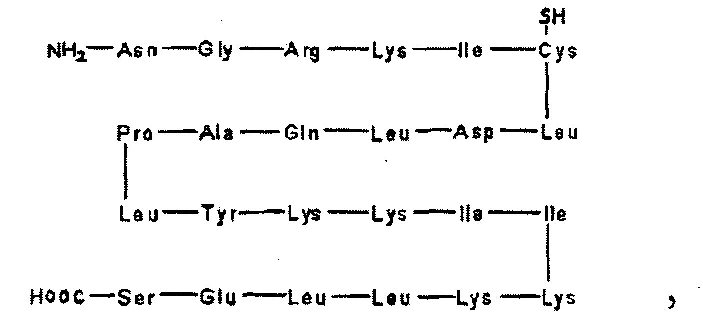

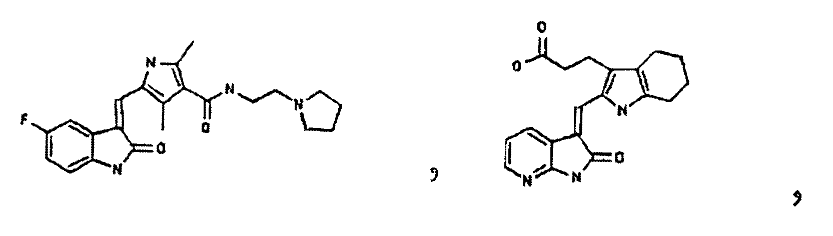

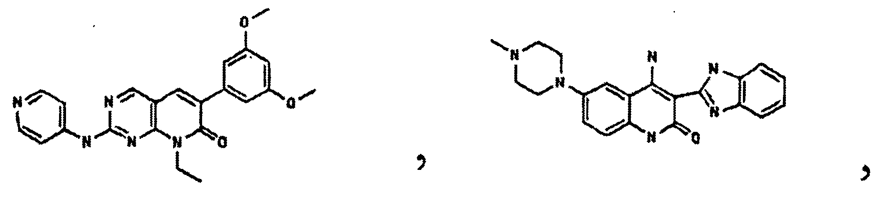

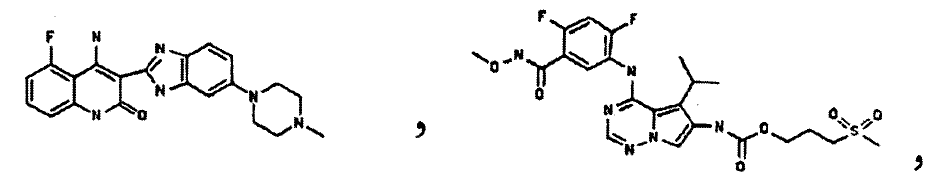

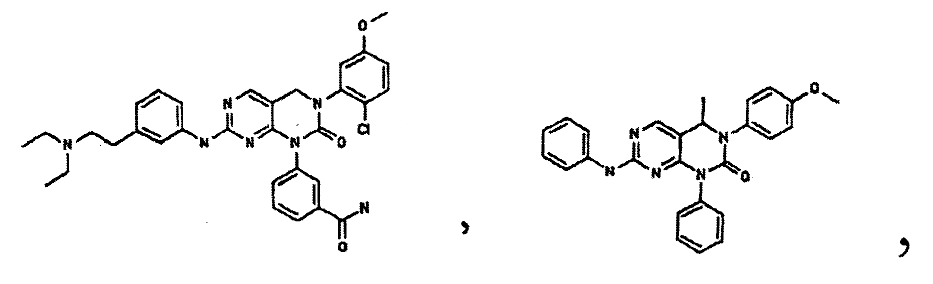

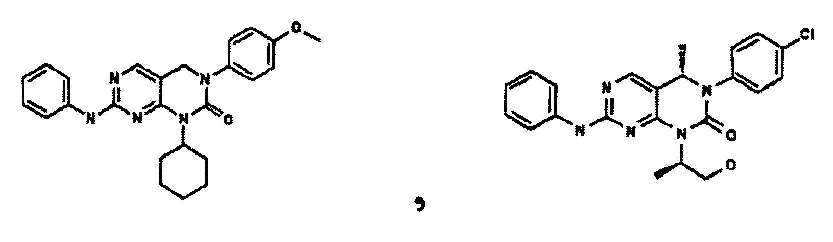

Alternatively, a ligand known to have affinity for FGFR can also be used as a test compound.

As used herein "ligand" means any substance accepted by FGFR, and includes both endogenous ligands essentially present in the FGFR-expressing organism and artificially prepared substance. As potential candidates of the ligand for FGFR, the substances given as examples below can be mentioned.

"Fibroblast growth factor (FGF)" is the family of polypeptides having strong growth-promoting activity on fibroblasts, and is a known ligand for FGFR.

As members of this family, FGF1 to FGF23 are known, and commercial products thereof (for example, commercially available from Pepro Tech Inc. and the like) can be used. Furthermore, a fragment of FGF can also be a candidate for the ligand for FGFR, as long as it has a binding site to FGFR.

Although the type of FGF used in the present invention is not subject to limitation, FGF-2, FGF-7, FGF-8, and FGF-17 are preferable, and FGF-2 and FGF-7 are particularly preferable.

An antibody against FGFR can also be a candidate for the ligand for FGFR. This antibody may be a polyclonal antibody or a monoclonal antibody, and can be prepared by an immunological technique widely known in the relevant field as exemplified below.

Furthermore, a fragment of an anti-FGFR antibody can also be a candidate for the ligand for FGFR, as long as it has an antigen binding site (CDR) to FGFR. As examples of the fragment of the antibody, Fab, F (ab')₂, ScFv, minibody and the like can be mentioned.

The polyclonal antibody can be acquired by, for example, administering FGFR or a fragment thereof (as required, may be prepared as a complex crosslinked to a carrier protein such as bovine serum albumin or KLH (Keyhole Limpet Hemocyanin)) as the antigen, along with a commercially available adjuvant (for example, Freund's complete or incomplete adjuvant), to an animal subcutaneously or intraperitoneally about 2 to 4 times at intervals of 2 to 3 weeks (the antibody titer of partially drawn serum is to be determined by a known antigen-antibody reaction and its elevation confirmed in advance), collecting whole blood about 3 to about 10 days after final immunization, and purifying the antiserum. As the animal to receive the antigen, mammals such as rats, mice, rabbits, goat, guinea pigs, and hamsters can be mentioned.

The monoclonal antibody can be prepared by, for example, a cell fusion method (for example, Takeshi Watanabe, Saibou Yugouhou No Genri To Monokuronaru Koutai No Sakusei, edited by Akira Taniuchi and Toshitada Takahashi, "Monokuronaru Koutai To Gan - Kiso To Rinsho -", pages 2-14, Science Forum Shuppan, 1985). For example, the factor, along with a commercially available adjuvant, is administered subcutaneously or intraperitoneally to a mouse 2 to 4 times, and about 3 days after final administration, the spleen or lymph nodes are collected, and leukocytes are collected. These leukocytes and myeloma cells (for example, NS-1, P3X63Ag8 and the like) are fused to obtain a hybridoma that produces a monoclonal antibody against the factor. This cell fusion may be performed by the PEG method [J. Immunol. Methods, 81(2): 223-228 (1985)], or by the electric pulse method [Hybridoma, 7(6): 627-633 (1988)]. A hybridoma that produces the desired monoclonal antibody can be selected by detecting an antibody that binds specifically to the antigen from the culture supernatant using a widely known EIA or RIA method and the like. Cultivation of the hybridoma that produces the monoclonal antibody can be performed in vitro, or in vivo such as in mouse or rat ascitic fluid, preferably in mouse ascitic fluid, and the antibody can be acquired from the culture supernatant of the hybridoma and the ascitic fluid of the animal, respectively.

Considering the therapeutic effects and safety in humans, the anti-FGFR antibody used in the present invention is preferably a chimeric antibody of a human and another animal (for example, mouse and the like), more preferably a humanized antibody, and particularly preferably a complete human antibody. Here, "chimeric antibody" refers to an antibody having an immunized animal-derived variable region (V region) and a human-derived constant region (C region), and "humanized antibody" refers to an antibody wherein all regions other than CDR are replaced with a human antibody. A chimeric antibody and humanized antibody can be acquired by, for example, cleaving out the sequence that encodes the V region or CDR from a mouse monoclonal antibody gene prepared by the same method as described above, cloning a chimeric gene prepared by fusion of the sequence with the DNA that encodes the C region of a human myeloma-derived antibody into an appropriate expression vector, and transfecting this into an appropriate host cell to allow the chimeric gene to be expressed. While a complete human antibody can be produced from a human-human (or mouse) hybridoma, for stable and economical provision of the antibody in large amounts, human antibody producing animals (e.g., mouse) (e.g.,: those manufactured by Abgenix [trade name: XenoMouose (see Nat. Genet., 15: 146-156, 1997; US Patent No. 5,939,598 and the like)], those manufactured by Medarex [trade name: Hu-Mab Mouse (see Nat. Biotechnol., 14: 845-851, 1996; US Patent No. 5,545,806 and the like)], those manufactured by Kirin Brewery Co., LTD. [trade name: KMmouse] etc.) or phage display method (for representative phage display library, library of CAT (see J. Mol. Biol., 222: 581-597, 1991; Nat. Biotechnol., 14: 309-314, 1996), library of MRC (see Annu. Rev. Immunol., 12: 433-455, 1994), library of Dyax (see J. Biol. Chem., 1999 (ibidem); Proc. Natl. Acad. Sci. USA, 14: 7969-7974, 2000), HuCAL library of Morphosys (see J. Mol. Biol., 296: 57-86, 2000), library of BioInvent (see Nat. Biotechnol., 18: 852, 2000), library of Crucell (see Proc. Natl. Acad. Sci. USA, 92: 3938, 1995; J. Immunol. Methods, 272: 219-233, 2003) and the like) are desirably used for its production.

Alternatively, as the antibody against FGFR, for example, an antibody commercially available from R&D Systems Inc. and the like can be used.

Affinity between a test compound and FGFR can be evaluated using a method of measuring receptor-ligand interactions widely known in the relevant field. For example, when a compound whose affinity for FGFR is a binding activity to the FGF-binding site is screened for, the compound acts in competition with FGF with respect to the binding to FGFR; therefore, for example, a method comprising labeling the free FGF described above with an appropriate labeling agent (for example, radioisotopes (e.g., [¹²⁵I], [¹³¹I], [³H], [¹⁴C] and the like), enzymes (e.g., β-galactosidase, β-glucosidase, alkaline phosphatase, peroxidase, malate dehydrogenase and the like), fluorescent substances (e.g., fluorescamine, fluorescein isothiocyanate and the like), luminescent substances (e.g., luminol, luminol derivatives, luciferin, lucigenin and the like) and the like are used), bringing solid-immobilized FGFR (e.g., FGFR-expressing cells, membrane fraction and the like adsorbed to a well plate) and a test compound into contact with each other in the presence of the labeled FGF, then removing unbound FGF by solid-liquid separation, and quantifying the amount (activity) of the labeling agent bound to the solid phase, and the like can be mentioned.

The hepatitis C virus (HCV) used in the present invention may be a naturally occurring virus, and may be an artificially engineered virus capable of mimicking the cell infection of HCV (for example, pseudotyped virus), or particles that have a surface structure similar to that of HCV but do not contain the genome (for example, HCV-LP (hepatitis C virus-like particles)).

As used herein, a pseudotyped virus of HCV is a recombinant virus capable of reflecting the cell infection of HCV, which has the extracellular domain of an envelop protein essential to the cell infection of HCV on the surface thereof, for example, a recombinant virus obtained by replacing at least the ectodomain of an envelope protein of other virus for which a reliable cell culture system has been established (e.g., vesicular stomatitis virus (VSV) and the like) with the ectodomains of two envelop proteins (E1 and E2) of HCV, and can be prepared by, for example, a method using a recombinant virus incorporating an HCV envelop protein in place of the original virus envelop protein on the virus genome, a method comprising infecting a cell to express the HCV envelop protein with a virus deprived of the original envelop protein to allow the virus to incorporate the HCV envelop protein upon budding (e.g., the method described in Virology Vol.286, pp.263-275, 2001 and the like) and the like.

As used herein, HCV-LP is a virus-like particle having the extracellular domain of the envelop protein essential to the cell infection of HCV on the surface thereof, for example, a virus-like particle obtained by separating and purifying a particle coated with a membrane having two envelop proteins of HCV (E1 and E2), secreted extracellularly or formed intracellularly in the form of vesicles or vacuoles in cells (e.g., insect cells) incorporating the envelop proteins, and can be prepared on the basis of, for example, the description in the Journal of Virology Vol.72, No.5, pp.3827-3836, 1998 and the like.

As the method of measuring the degree of the cell infection of the virus, a method widely known in the art can be used. For example, when the above-described pseudotyped virus is used as the HCV, by incorporating a reporter gene alongside, for example, the secretory alkaline phosphatase (SEAP) gene, GFP gene, luciferase gene, beta-galactosidase gene and the like, the degree of the cell infection of HCV can be measured with the expression level of the reporter gene in cells infected by the virus as the index.

For evaluation of the degree of the cell infection of the virus utilizing the above-described reporter gene, a commercial kit (commercially available from, for example, TOYOBO Co. Ltd., Clontech Inc., and the like) can be utilized.

The present invention also provides a kit for screening/identification suitable for embodying the above-described screening/identification method of the present invention. The kit comprises a lipid bilayer membrane comprising FGFR and/or FGFR-expressing cells, and a pseudotyped virus of HCV. Here, the lipid bilayer membrane may be a membrane fraction prepared from cells having FGFR, or may be a proteoliposome wherein FGFR is embedded in an artificial lipid bilayer membrane.

The present invention further relates to an inhibitor of the cell infection of HCV comprising a compound having affinity for FGFR, which can be obtained by the above-described screening/identification method.

In a preferred mode of embodiment, the above-described compound having affinity for FGFR has a binding activity to the FGF-binding site of FGFR.

The present invention further relates to an inhibitor of the cell infection of HCV comprising a compound having the capability of blocking the binding of FGFR and HCV, which can be obtained by the above-described screening/identification method.

In a preferred mode of embodiment, the above-described compound having the capability of blocking the binding of FGFR and HCV is the above-described compound having affinity for FGFR; in another preferred mode of embodiment, the above-described compound having the capability of blocking the binding of FGFR and HCV is the above-described solubilized FGFR.

The formulation (inhibitor) of the present invention may be formulated with an optional carrier, for example, a pharmaceutically acceptable carrier.

Here, as "a pharmaceutically acceptable carrier", an excipient, diluent, bulking agent, disintegrant, stabilizer, preservative, buffering agent, emulsifier, flavoring agent, colorant, sweetener, thickening agent, taste corrective, solubilizer or other additives and the like can be mentioned. By using one or more of such carriers, formulations in the form of tablets, pills, powders, granules, injections, solutions, capsules, troches, elixirs, suspensions, emulsions, syrups and the like can be prepared. These formulations can be administered orally or parenterally.

The dose of the formulation capable of inhibiting the cell infection of HCV varies depending on the recipient's symptoms, age, body weight, route of administration and the like; in the case of oral administration, for example, the dose is about 0.1 to about 10,000 mg, preferably about 0.1 to about 3,000 mg, more preferably about 0.1 to about 1,000 mg, per day for a patient (assuming a 60 kg body weight). On the other hand, in the case of parenteral administration, for example, as an injection, the dose is about 0.01 to about 5,000 mg, preferably about 0.01 to about 2,000 mg, more preferably about 0.01 to about 500 mg, per day for a patient (assuming a 60 kg body weight).

The present invention is explained in more detail by referring to the following Examples, which are mere examples, that should not be construed as limitative.

### Examples

### (Production Example 1: Preparation of a pseudotyped virus of HCV)

Preparation of a pseudotyped virus of HCV was performed according to the same method as described in Virology Vol.286, pp.263-275, 2001. First, on the basis of the method described in Proc. Natl. Acad. Sci. USA, Vol.92, pp.4477-4481, 1995, a recombinant virus having a genome wherein the envelop protein (glycoprotein) gene of vesicular stomatitis virus (VSV) is replaced with the secretory alkaline phosphatase (SEAP) or green fluorescent protein (GFP) gene, and coated with the glycoprotein of VSV (hereinafter one wherein the envelop protein (glycoprotein) gene is replaced with the SEAP gene is referred to as ΔG/SEAP/VSV G, and one wherein the envelop protein (glycoprotein) is replaced with the GFP gene is referred to as ΔG/GFP/VSV G) was prepared. Next, ΔG/SEAP/VSV G or ΔG/GFP/VSV G was infected to CHO-K1 cells having the glycoprotein gene of VSV expressed forcedly therein to prepare ΔG/SEAP/VSV G and ΔG/GFP/VSV G in large amounts, which were supplied as control viruses in the Examples below. Also, ΔG/SEAP/VSV G or ΔG/GFP/VSV G was infected to CHO-K1 cells having a chimeric protein consisting of ectodomains of the E1 and E2 protein of HCV and the C-terminal region of the glycoprotein of VSV, expressed forcedly therein, to prepare recombinant viruses having the SEAP or GFP gene in the virus genome, coated with the E1 and E2 proteins of HCV (hereinafter referred to as ΔG/SEAP/HCV E1E2 and ΔG/GFP/HCV E1E2, respectively) in large amounts, which were supplied as pseudotyped viruses in the Examples below.

### (Production Example 2: Preparation of HCV-LP)

Preparation of HCV-LP was performed according to the same method as described in Journal of Virology Vol.72, No.5, pp.3827-3836, 1998. As the HCV cDNA for incorporation in the baculovirus vector, position 330 to 2849 of the base sequence of the HCV J1 genome shown by SEQ ID NO:1 (corresponding to the amino acid for position 1 to 836 of HCV polyprotein) was used.

### (Production Example 3: Preparation of FGFR-5/Fc)

FGFR-5/Fc, which is a chimeric protein prepared by fusing the Fc region of human immunoglobulin to the C-terminal side of the extracellular domain of human FGFR-5, via a linker polypeptide comprising the sequence of Factor Xa (consisting of the amino acid sequence shown by SEQ ID NO:5) was used as the solubilized FGFR-5 in the Examples below. Preparation of this FGFR-5/Fc was performed according to a conventional method as described below. The expression vector pME18S (see Maruyama et al., Medical Immunology, 20(1) pp.27-32 (1990)), which incorporated the DNA that encodes FGFR-5/Fc (consisting of the DNA that encodes the extracellular domain of human FGFR-5 (SEQ ID NO:2)/the DNA that encodes the linker polypeptide (SEQ ID NO:4)/the DNA that encodes the Fc region of human immunoglobulin (SEQ ID NO:3)) was transfected into HEK293F cells, and these cells were cultured to allow the FGFR-5/Fc protein to be expressed in a large amount. Next, the culture supernatant containing the expression product was collected and purified using a Protein A column, whereby FGFR-5/Fc was acquired.

### (Example 1: Effects of various members of the fibroblast growth factor (FGF) family on the infectivity of a pseudotyped virus of HCV to cells)

(Materials)
cell: HepG2
virus: ΔG/SEAP/HCV E1E2, ΔG/SEAP/VSV G
reagent: D-MEM high glucose (Nikken Bio Medical Laboratory), FBS (CTT), Reporter Assay Kit-SEAP- (TOYOBO SAK-101 Lot. 316100), FGF-1, FGF-2, FGF-4, FGF-5, FGF-6, FGF-7, FGF-8, FGF-9, FGF-10, FGF-16, FGF-17, FGF-18, FGF-19, FGF-20, EGF, HGF (Pepro Tech)

### (Method)

HepG2 cells were seeded at 0.8 x 10⁴ cells/well/80 µl/96 wp, after which they were cultured overnight (O/N) at 37°C in the presence of 5% CO₂. Each of the above-described human-derived FGFs, and epithelial growth factor (EGF) and hepatocyte growth factor (HGF) were prepared at 100 µg/ml according to the instructions of the above-described Reporter Assay Kit, after which they were serially diluted with PBS, and 10 µl aliquots were added to the cells. After a reaction at 37°C in the presence of 5% CO₂ for 1 hour, 1 x 10¹⁰ genome/10 µl of ΔG/SEAP/HCV E1E2 or 1 x 10⁸ genome/10 µl of ΔG/SEAP/VSV G was added, and the cells were cultured at 37°C in the presence of 5% CO₂ for 24 hours. To measure the amount of SEAP secreted by the infecting virus into the culture supernatant, 10 µl of the medium was transferred to Corning ® 96 Well Black Round Bottom Polystyrene Not Treated Microplate, 25 per Bag, without Lid, Non-Sterile (Cat. #3792), 10 µl of the inhibitory solution of the above-described Reporter Assay Kit was added, and the reaction was allowed to proceed at room temperature (RT) for 30 minutes. Next, 80 µl of a luminescent reagent was added, and the reaction was allowed to proceed at 37°C for 30 minutes, after which a measurement was performed using a luminometer. From the values obtained, the IC₅₀ value for each FGF was calculated with the SEAP amount for cells containing none of the growth factors as 100%.

### (Results and discussion)

As a result of this experiment, it was found that FGF-2, FGF-7, FGF-8, and FGF-17 inhibited the infection of ΔG/SEAP/HCV E1E2, with the inhibition by FGF-2 and FGF-7 being particularly remarkable (Table 1). From this fact, it was suggested that FGFR might be involved in the cell infection (cell adsorption and/or entry into the cell) of HCV, and that FGFs (particularly FGF-2, FGF-7, FGF-8 and FGF-17; more particularly FGF-2 and FGF-7) might inhibit the above-described cell infection of HCV medicated by FGFR. Therefore, it was suggested that (i) an assay system for evaluating the degree of cell infection using natively FGFR-expressing cells (for example, HepG2) and a pseudotyped virus of HCV could be utilized to select or judge a compound capable of inhibiting the cell infection of HCV from compounds having affinity for FGFR or the capability of blocking the binding of FGFR and HCV, that (ii) by evaluating the degree of competition with FGF in the assay system, a compound having affinity for FGFR or the capability of blocking the binding of FGFR and HCV, and capable of inhibiting the cell infection of HCV, could be screened for or identified, and that (iii) these compounds selected or judged or screened for or identified (including FGF) could be utilized as inhibitors of the cell infection of HCV in the prevention or treatment of hepatitis C.

**Table 1**

| | IC₅₀ | |
|---|---|---|
| | ΔG/SEAP/HCV E1E2 | ΔG/SEAP/VSV G |
| human FGF 1 (acidic) | - | - |
| human FGF 2 (basic) | 1.36 | - |
| human FGF 4 | - | - |
| human FGF 5 | - | - |
| human FGF 6 | - | - |
| human FGF 7 (KGF) | 2.32 | - |
| human FGF 8 | 8.51 | - |
| human FGF 9 | - | - |
| human FGF 10 | >10 | - |
| human FGF 16 | - | - |
| human FGF 17 | 7.41 | - |
| human FGF 18 | >10 | - |
| human FGF 19 | - | - |
| human FGF 20 | >10 | - |
| human EGF | - | - |
| human HGF | - | - |
| | | (µg/ml) |

### (Example 2: Infectivity of a pseudotyped virus of HCV to cells having FGFR expressed forcedly therein) (Materials)

cell: CHO-K1
virus: ΔG/SEAP/HCV E1E2, ΔG/SEAP/VSV G
reagent: D-MEM high glucose (Nikken Bio Medical Laboratory), FBS (CTT), Reporter Assay Kit-SEAP-(TOYOBO SAK-101 Lot.316100), G418(PAA lab.)
(Equipment)
FACSCalibur (Becton Dickinson)

### (Method)

A cDNA of human-derived FGFR-4 corresponding to position 157 to 2565 of the mRNA sequence shown by GenBank accession number: NM_002011, and a cDNA of FGFR-5 corresponding to position 23 to 1537 of the mRNA sequence shown by GenBank accession number: NM_021923, cloned by RT-PCR, were inserted and subcloned into the HindIII-XbaI site of the expression vector pcDNA 3.1(-) (Invitrogen) and the BamHI-EcoRI site of the expression vector pEF1/MycHisA (Invitrogen), respectively, and CHO-K1 cells were transfected with each vector obtained, subjected to selection culture with G418 (3 mg/ml), and maintained, as required, further selected using a cell sorter, whereby respective CHO cells having FGFR expressed forcedly therein (CHO/FGFR-4 and CHO/FGFR-5) were obtained. These, along with CHO-K1 cells transfected with pcDNA3.1 and non-transfected CHO-K1 cells, were each seeded at 8 x 10³ cells/well/90 µl/96 wp, after which they were cultured (O/N) at 37°C in the presence of 5% CO₂. 1 x 10¹⁰ genome/10 µl of ΔG/SEAP/HCV E1E2 or 1x10¹⁰ genome/10 µl of ΔG/SEAP/VSV G was infected to the cells, and adsorbed at 37°C in the presence of 5% CO₂ for 1 hour, after which the cells were once washed with the medium, and the cells were cultured at 37°C in the presence of 5% CO₂ for 24 hours. To measure the SEAP amount in the culture supernatant, 10 µl of the medium was transferred to Corning ® 96 Well Black Round Bottom Polystyrene Not Treated Microplate, 25 per Bag, without Lid, Non-Sterile (Cat. #3792), 10 µl of the inhibitor solution in the Reporter Assay Kit (TOYOBO SAK-101 Lot.316100) was added, and the reaction was allowed to proceed at room temperature (RT) for 30 minutes. Next, 80 µl of a luminescent reagent was added, and the reaction was allowed to proceed at 37°C for 30 minutes, after which SEAP amounts were measured using a luminometer. From the values obtained, the relative value of SEAP amount for each type of cells was calculated with the SEAP amount for non-transfected cells as 100.

### (Results and discussion)

As a result of this experiment, as shown in Fig. 1, in CHO/FGFR-4, the promoting activity on the infectivity of ΔG/SEAP/HCV E1E2 was very low. By contrast, in CHO/FGFR-5, the infectivity of ΔG/SEAP/HCV E1E2 rose about 6 times compared to the control. From this fact, it was suggested that FGFR-5 might be involved in the entry into the cell of HCV. Therefore, it was suggested that an assay system for evaluating the degree of the cell infection using cells (for example, CHO) having FGFR, particularly FGFR-5 involved in the entry into the cell, expressed forcedly therein, and a pseudotyped virus of HCV, could be utilized for screening or identification of a compound having affinity for FGFR or the capability of blocking the binding of FGFR and HCV, and capable of inhibiting the cell infection of HCV.

### (Example 3: Binding activity of HCV-LP to cells having FGFR expressed forcedly therein)

### (Materials)

cell: CHO-K1
virus: HCV-LP reagent: D-MEM high glucose (Nikken Bio Medical Laboratory), FBS (CTT), PBS (-) (Nikken Bio Medical Laboratory), G418 (PAA lab.), Core ELISA kit (Ortho-Clinical Diagnostics)
(Equipment)
FACSCalibur (Becton Dickinson)

### (Method)

By the same method as Example 2, CHO cells having FGFR expressed forcedly therein (CHO/FGFR-4 and CHO/FGFR-5) were obtained. The CHO/FGFR-4 and CHO/FGFR-5 obtained and non-transfected CHO-K1 cells were each seeded at 5 x 10⁴ cells/well/280 µl/48 wp, after which they were cultured (O/N) at 37°C in the presence of 5% CO₂. HCV-LP equivalent to 30 pg/20 µl of HCV core protein (quantified using the Ortho HCV antigen ELISA test (pharmaceutical for extracorporeal diagnosis; approval number 21400AMZ00485000)) was inoculated, and adsorption was performed at 4°C for 2 hours, after which the cells were washed with PBS 3 times, and the cells were recovered. The amount of HCV-LP adsorbed to the recovered cells was measured using a core ELISA kit according to the instructions attached to the kit.

### (Results and discussion)

As a result of this experiment, as shown in Fig. 2, HCV-LP bound specifically to CHO/FGFR-4 and CHO/FGFR-5. From this fact, it was suggested that FGFR-4 and FGFR-5 might be involved in the cell adsorption of HCV. Therefore, it was suggested that an assay system for evaluating the binding activity of cells (for example, CHO) having FGFR (particularly FGFR-4 or FGFR-5) expressed forcedly therein and HCV-LP using both of them could be utilized for screening or identification of a compound having affinity for FGFR or the capability of blocking the binding of FGFR and HCV, and capable of inhibiting the cell infection of HCV.

### (Example 4: Binding activity of HCV in patient plasma to cells having FGFR expressed forcedly therein)

### (Materials)

cell: CHO-K1
virus: window period HCV patient plasma (Type 1a) (BioClinical Partners, Inc.)
reagent: D-MEM high glucose (Nikken Bio Medical Laboratory), FBS (CTT), PBS(-) (Nikken Bio Medical Laboratory), G418 (PAA lab.), TaqMan (Applied Biosystems)
(Equipment)
ABI PRISM 7000 (Applied Biosystems)

### (Method)

By the same method as Example 2, CHO cells having FGFR expressed forcedly therein (CHO/FGFR-4 and CHO/FGFR-5) were obtained. The CHO/FGFR-4 and CHO/FGFR-5 obtained and non-transfected CHO-K1 cells were each seeded at 1 x 10⁵ cells/well/500 µl/24 wp, after which they were cultured (O/N) at 37°C in the presence of 5% CO₂. HCV patient plasma was inoculated to each cell, and the cells were cultured at 37°C for 24 hours, after which the cells were washed with PBS(-) 3 times. The RNA amount of HCV bound to each cell was quantified by real-time PCR.

### (Results and discussion)

As shown in Fig. 3, HCV in patient plasma bound specifically to CHO/FGFR-4 and CHO/FGFR-5. From this fact, it was suggested that FGFR-4 and FGFR-5 might be a host factor that binds to HCV. Therefore, it was suggested that an assay system for evaluating the binding activity of cells having FGFR (particularly FGFR-4 or FGFR-5) expressed forcedly therein (for example, CHO) and patient-derived HCV using both of them could be utilized for screening or identification of a compound having affinity for FGFR or the capability of blocking the binding of FGFR and HCV, and capable of inhibiting the cell infection of HCV.

### (Example 5: Infectivity of a pseudotyped virus of HCV to FGFR knockdown cells)

### (Materials)

cell: Huh7
virus: ΔG/GFP/HCV E1E2, ΔG/GFP/VSV-G
reagent: D-MEM high glucose (Nikken Bio Medical Laboratory), OPTI-MEM (Invitrogen), FBS (CTT), control siRNA, FGFR-4 siRNA, FGFR-5 siRNA (B-Bridge), siFECTOR (B-Bridge)
(Equipment)
FACSCalibur (Becton Dickinson)

### (Method)

Huh7 cells were seeded at 4 x 10⁵ cells/well/2 ml/6 wp, after which they were cultured (O/N) at 37°C in the presence of 5% CO₂, A siRNA that knocks down FGFR-4, a siRNA that knocks down FGFR-5, and a siRNA that does not knock down FGFR as the control, were prepared at a final concentration of 40 nM and/or 80 nM according to the instructions of siFECTOR, and cultured Huh7 cells were transfected therewith. Each transfected Huh7 cell and non-transfected Huh7 cells as the control were cultured at 37°C in the presence of 5% CO₂ for 24 hours, after which they were re-seeded and cultured at 37°C in the presence of 5% CO₂ for 24 hours, after which ΔG/GFP/HCV E1E2 or ΔG/GFP/VSV-G was inoculated at a m.o.i. (multiplicity of infection) of 0.1. Furthermore, the cells were cultured at 37°C in the presence of 5% CO₂ for 24 hours, GFP-positive cells were counted by FACS, and their positivity rate was calculated with the GFP amount for non-transfected cells as 100%.

### (Results and discussion)

As a result of this experiment, as shown in Fig. 4, it was found that when the FGFR-4 of Huh7 cells was knocked down, the infectivity of ΔG/GFP/HCV E1E2 was inhibited dependently on the concentration of siRNA. Also, in the FGFR-5 knockdown Huh7 cells, in the presence of the same concentration of siRNA, more potent inhibition of infection was observed than in the FGFR-4 knockdown cells. From this fact, it was suggested that FGFR-4 might not be positively involved in the entry into the cell of HCV but might play a certain role in the cell adsorption. Also, it was suggested that FGFR-5 might be involved in the cell adsorption and entry into the cell of HCV.

### (Example 6: Effects of solubilized FGFR-1 to FGFR-4 on the infectivity of a pseudotyped virus of HCV to cells)

### (Materials)

cell: HepG2
virus: ΔG/SEAP/HCV E1E2, ΔG/SEAP/VSV G
reagent: D-MEM high glucose(Nikken Bio Medical Laboratory), FBS (CTT), Reporter Assay Kit -SEAP-(TOYOBO SAK-101 Lot. 316100), FGFR-1α IIIb/Fc, FGFR-1β IIIb/Fc, FGFR-1α IIIc/Fc, FGFR-1β IIIc/Fc, FGFR-2α IIIb/Fc, FGFR-2β IIIb/Fc, FGFR-2α IIIc/Fc, FGFR-2β IIIc/Fc, FGFR-3α IIIc/Fc, FGFR-4/Fc (SIGMA)

### (Method)

HepG2 cells were seeded at 8 x 10³ cells/well/80 µl/96 wp, after which they were cultured (O/N) at 37°C in the presence of 5% CO₂. To 10 µl of each FGFR/Fc as the solubilized FGFR prepared at 100 µg/ml according to the instructions of the above-described Reporter Assay Kit and then serially diluted with PBS, 1 x 10⁹ genome/10 µl of ΔG/SEAP/HCV E1E2 or 1 x 10⁷ genome/10 µl of ΔG/SEAP/VSV G was added; the reaction was allowed to proceed at 37°C in the presence of 5% CO₂ for 1 hour. Each FGFR/Fc-virus mixture and each virus alone as the control were each inoculated to cultured HepG2 cells, and adsorption was performed at 37°C in the presence of 5% CO₂ for 1 hour, after which the cells were twice washed with the culture broth. The cells were cultured at 37°C in the presence of 5% CO₂ for 24 hours, after which the SEAP amount in each culture supernatant was measured, and from the values obtained, the relative infection rate (%) of each FGFR/Fc-treated cell was calculated with the infection level of cells inoculated with ΔG/SEAP/HCV E1E2 alone as 100%.

### (Results and discussion)

As a result of this experiment, as shown in Fig. 5, by solubilized FGFR-1β IIIb, FGFR-1β IIIc, FGFR-2β IIIb, FGFR-2α IIIc, FGFR-4, the infectivity of ΔG/SEAP/HCV E1E2 was inhibited. From this fact, it was suggested that FGFR-1 to FGFR-4 (particularly FGFR-4) might be involved in the cell infection of HCV (at least cell adsorption or entry into the cell), and that solubilized FGFR-1 to FGFR-4 (particularly FGFR-4) might inhibit the above-described cell infection of HCV mediated by FGFR-1 to FGFR-4 (particularly FGFR-4). Therefore, it was suggested that (i) an assay system for evaluating the degree of competition of solubilized FGFR for the cell infection using natively FGFR-expressing cells (for example, HepG2), a pseudotyped virus of HCV and solubilized FGFR could be utilized for screening or identification of a compound having affinity for FGFR or the capability of blocking the binding of FGFR and HCV, and capable of inhibiting the cell infection of HCV, and that (ii) solubilized FGFR per se could also be utilized as an inhibitor of the cell infection of HCV in the prevention or treatment of hepatitis C.

### (Example 7: Effects of solubilized FGFR-5 on the infectivity of a pseudotyped virus of HCV to cells)

### (Materials)

cell: HepG2
virus: ΔG/SEAP/HCV E1E2, ΔG/SEAP/VSV G
reagent: D-MEM high glucose(Nikken Bio Medical Laboratory), FBS (CTT), Reporter Assay Kit -SEAP-(TOYOBO SAK-101 Lot. 316100), FGFR-5/Fc

### (Method)

HepG2 cells were seeded at 8 x 10³ cells/well/80 µl/96 wp, after which they were cultured (O/N) at 37°C in the presence of 5% CO₂. Solubilized FGFR-5 (FGFR-5/Fc) prepared according to Production Example 3 was prepared at 100 µg/ml, after which it was serially diluted with PBS. Next, for the series of concentrations of FGFR-5/Fc prepared by the dilution, (A) 10 µl of FGFR-5/Fc and 1 x 10⁹ genome/10 µl of ΔG/SEAP/HCV E1E2 or 1 x 10⁷ genome/10 µl of ΔG/SEAP/VSV G were mixed, and the reaction was allowed to proceed at 37°C in the presence of 5% CO₂ for 1 hour, after which each FGFR-5/Fc-virus mixture was inoculated to cultured HepG2 cells, or (B) 10 µl of FGFR-5/Fc and 1 x 10⁹ genome/10 µl of ΔG/SEAP/HCV E1E2 or 1 x 10⁷ genome/10 µl of ΔG/SEAP/VSV G were mixed and at the same time inoculated to cultured HepG2 cells, or (C) 1 x 10⁹ genome/10 µl of ΔG/SEAP/HCV E1E2 or 1 x 10⁷ genome/10 µl of ΔG/SEAP/VSV G was inoculated to cultured HepG2 cells, and the cells were cultured at 37°C in the presence of 5% CO₂ for 1 hour, after which 10 µl of FGFR-5/Fc was added. For control, each virus alone was inoculated to cultured HepG2 cells. These cells were cultured at 37°C in the presence of 5% CO₂ for 24 hours, after which the SEAP amount in each culture supernatant was measured according to the instructions of the Reporter Assay Kit, and the inhibition rate (%) for the cell infection of the virus with each concentration of FGFR-5/Fc was calculated with the SEAP amount for cells inoculated with the virus alone as the reference.

### (Results and discussion)

As a result of this experiment, as shown in Fig. 6, by FGFR-5/Fc, the infection of ΔG/SEAP/HCV E1E2 to HepG2 cells was inhibited dose-dependently. FGFR-5/Fc exhibited a more potent infection inhibitory effect at lower FGFR/Fc concentrations than the other FGFR/Fcs, including FGFR-4/Fc in Example 6 when inoculated to the cells after being mixed with the virus ((A) above). Also, when added after the virus alone was inoculated to the cells ((C) above), FGFR-5/Fc still significantly inhibited the infection of ΔG/SEAP/HCV E1E2 to HepG2 cells, although the infection inhibitory effect thereof decreased, compared to the case where it was inoculated to the cells after being mixed with the virus. From this fact, it was suggested that FGFR-5 might be involved in the cell infection (particularly entry into the cell) of HCV, and that solubilized FGFR-5 might potently inhibit the above-described cell infection of HCV mediated by FGFR-5. Therefore, it was suggested that solubilized FGFRs, particularly solubilized FGFR-5, could be utilized as an inhibitor of the cell infection of HCV in the prevention or treatment of hepatitis C.

### (Example 8: Effects of anti-mouse FGFR-5 polyclonal antibody on the infectivity of a pseudotyped virus of HCV to HepG2 cells)

### (Materials)

cell: HepG2
virus: ΔG/SEAP/HCV E1E2, ΔG/SEAP/VSV G
reagent: D-MEM high glucose(Nikken Bio Medical Laboratory), FBS (CTT), Reporter Assay Kit -SEAP-(TOYOBO SAK-101 Lot. 316100), anti-mouse FGFR-5 polyclonal antibody (R&D Systems Catalog No. AF1899)

### (Method)

HepG2 cells were seeded at 0.8 x 10⁴ cells/well/80 µl/96 wp, after which they were cultured (O/N) at 37°C in the presence of 5% CO₂. An anti-mouse FGFR-5 polyclonal antibody was prepared at 300 µg/ml, after which it was serially diluted with PBS, and 10 µl of each dilution was added. After a reaction at 37°C in the presence of 5% CO₂ for 1 hour, 1 x 10¹⁰ genome/10 µl of ΔG/SEAP/HCV E1E2 or 1 x 10⁸ genome/10 µl of ΔG/SEAP/VSV G was added, and the cells were cultured at 37°C in the presence of 5% CO₂ for 24 hours. The SEAP amount in each culture supernatant was measured according to the instructions of the Reporter Assay Kit, and the inhibition rate (%) for the infection of ΔG/SEAP/HCV E1E2 and ΔG/SEAP/VSV G to the cells by the anti-mouse FGFR-5 antibody was calculated with the SEAP amount for cells inoculated with each virus alone as the reference.

### (Results and discussion)

As a result of this experiment, as shown in Fig. 7, by the anti-mouse FGFR-5 antibody, the infection of ΔG/SEAP/HCV E1E2 to the cells was inhibited dose-dependently. From this fact, it was suggested that a compound having affinity for FGFR or the capability of blocking the binding of FGFR and HCV (for example, an antibody having affinity for FGFR-5) could be utilized as an inhibitor of the cell infection of HCV in the prevention or treatment of hepatitis C.

### (Example 9: Effects of solubilized FGFR-5 on the binding activity of HCV in patient plasma to cells)

### (Materials)

cell: HepG2 and Huh7
virus: window period HCV patient plasma (Type 1a) (BioClinical Partners, Inc.)
reagent: D-MEM high glucose (Nikken Bio Medical Laboratory), FBS (CTT), PBS(-) (Nikken Bio Medical Laboratory), TaqMan (Applied Biosystems), FGFR-5/Fc
(Equipment)
ABI PRISM 7000 (Applied Biosystems)

### (Method)

HepG2 and Huh7 cells were respectively seeded at 1 x 10⁵ cells/well/500 µl/24 wp, after which they were cultured (O/N) at 37°C in the presence of 5% CO₂. Solubilized FGFR-5 (FGFR-5/Fc) prepared according to Production Example 3 was prepared at 100 µg/ml, after which it was serially diluted with PBS. Next, 10 µl of each of the series of concentrations of FGFR-5/Fc prepared by the dilution and HCV patient plasma comprising HCV genomic RNA (1 x 10⁶ copies/5 µl) were mixed in the medium to 500 µl, and the reaction was allowed to proceed at 37°C in the presence of 5% CO₂ for 1 hour. Also, for control, 500 µl of a medium containing only HCV patient plasma comprising HCV genomic RNA (1 x 10⁶ copies/5 µl) was prepared. Each FGFR-5/Fc-HCV patient plasma mixture and the control were respectively inoculated to each cell by exchanging the medium, and the cells were cultured at 37°C for 24 hours, after which the cells were washed with PBS(-) 3 times. The RNA amount of HCV bound to each cell was quantified by real-time PCR.

### (Results and discussion)

As a result of this experiment, as shown in Fig. 8, by FGFR-5/Fc, the binding of HCV in patient plasma to HepG2 and Huh7 cells was inhibited dose-dependently. From this fact, it was suggested that FGFR-5 might be involved in the cell infection (at least cell adsorption) of HCV, and that solubilized FGFR-5 might potently inhibit the above-described cell infection of HCV mediated by FGFR-5. Therefore, it was suggested that (i) an assay system for evaluating the degree of competition of solubilized FGFR for the cell infection using natively FGFR-expressing cells (for example, HepG2 and Huh7), patient-derived HCV and solubilized FGFR could be utilized for screening or identification of a compound having affinity for FGFR or the capability of blocking the binding of FGFR and HCV, and capable of inhibiting the cell infection of HCV, and that (ii) a compound having the capability of blocking the binding of FGFR and HCV, and inhibiting the cell infection of HCV (for example, solubilized FGFR, particularly solubilized FGFR-5) could be utilized as an inhibitor of the cell infection of HCV in the prevention or treatment of hepatitis C.

### (Example 10: Effects of solubilized FGFR-5 on the infectivity of a pseudotyped virus of HCV to human hepatocyte primary culture)

### (Materials)

cell: human hepatocyte primary culture
virus: ΔG/SEAP/HCV E1E2, ΔG/SEAP/VSV G
reagent: D-MEM high glucose(Nikken Bio Medical Laboratory), FBS (CTT), Reporter Assay Kit -SEAP-(TOYOBO SAK-101 Lot. 316100), FGFR-5/Fc

### (Method)

Human hepatocyte primary cultures were seeded at 8 x 10³ cells/well/80 µl/96 wp, after which they were cultured (O/N) at 37°C in the presence of 5% CO₂. Solubilized FGFR-5 (FGFR-5/Fc) prepared according to Production Example 3 was prepared at 100 µg/ml, after which it was serially diluted with PBS. Next, 10 µl of each of the series of concentrations of FGFR-5/Fc prepared by the dilution and 1 x 10⁹ genome/10 µl of ΔG/SEAP/HCV E1E2 or 1 x 10⁷ genome/10 µl of ΔG/SEAP/VSV G were mixed, and the reaction was allowed to proceed at 37°C in the presence of 5% CO₂ for 1 hour. Each FGFR-5/Fc-virus mixture and each virus alone as the control were inoculated to cultured cells. These cells were cultured at 37°C in the presence of 5% CO₂ for 24 hours, after which the SEAP amount in each culture supernatant was measured according to the instructions of the Reporter Assay Kit, and the virus infection rate (%) for cells treated at each concentration of FGFR-5/Fc was calculated with the SEAP amount for cells inoculated with the virus alone as the reference.

### (Results and discussion)

As a result of this experiment, as shown in Fig. 9, by FGFR-5/Fc, the infection of ΔG/SEAP/HCV E1E2 to human hepatocyte primary culture was inhibited dose-dependently. From this fact, it was suggested that FGFR-5 might be involved in the cell infection (particularly entry into the cell) of HCV, and that solubilized FGFR-5 might potently inhibit the above-described cell infection of HCV mediated by FGFR-5. Therefore, it was suggested that (i) an assay system for evaluating the degree of competition of solubilized FGFR for the cell infection using natively FGFR-expressing cells (for example, human hepatocyte primary culture), a pseudotyped virus of HCV and solubilized FGFR could be utilized for screening or identification of a compound having affinity for FGFR or the capability of blocking the binding of FGFR and HCV, and capable of inhibiting the cell infection of HCV, and that (ii) a compound having the capability of blocking the binding of FGFR and HCV, and inhibiting the cell infection of HCV (solubilized FGFR, particularly solubilized FGFR-5)could be utilized as an inhibitor of the cell infection of HCV in the prevention or treatment of hepatitis C.

### (Production Example 4: Preparation of anti-FGFR-5 monoclonal antibody)

### (1) Construction of FLAG-FGFR5 expression vector

(Materials)
reagent: pEF1/FGFR5;
primer (JBioS)
1. TAA TAC GAC TCA CTA TAG GGA GAC CCA AGC TGG CTA GTT AAG CTT GGT A (SEQ ID NO: 6)
2. TTT ATC GTC ATC GTC TTT GTA GTC CTT TGG GGG GCC TCG GGC GGC GGC G (SEQ ID NO: 7)
3. GAC TAC AAA GAC GAT GAC GAT AAA ATG GCG GAC AAG GTG GTC CCA CGG C (SEQ ID NO: 8)
4. GCT TGG TAC CGA GCT CGG ATC CGC (SEQ ID NO: 9)
   * Underlined part shows FLAG sequence to be newly inserted;
   KOD-plus polymerase (TOYOBO Ca#KOD-201);
   GeneAmp PCR System 9600 (Perkin Elmer);
   QIAquick gel extraction kit (QIAGEN Ca#28704);
   Klenow fragment (Takara Ca#2114A);
   QIAquick nucleotide removal kit (QIAGEN Ca#28304);
   BamHI (TOYOBO);
   ClaI (Takara);
   DNA ligation kit (Takara Ca#6022);
   JM109 (Takara Ca#9052);
   Endofree Plasmid Maxi kit (QIAGEN Ca#12362)

### (Method)

An expression vector having FLAG inserted between lysine at position 30 and methionine at position 31, located at the N-terminus (C-terminus of the signal sequence) of FGFR5 (GenBank accession number: NM_021923; SEQ ID NO:22 and 23), was prepared.

As the source for the FGFR5 gene, the pEF1/FGFR5 plasmid prepared by cloning the full-length FGFR5 sequence into the BamHI and EcoRI sites of the pEF1/mysHisA vector (Invitrogen) was used.

The sequence of FGFR5 on the N-terminal side from the FLAG insertion site by a combination of primers 1 (forward) and 2 (reverse), and the sequence of FGFR5 on the C-terminal side from the FLAG insertion site by a combination of primers 3 (forward) and 4 (reverse), were amplified by PCR in a way such that the FLAG sequence was added by primers 2 and 3. The PCR reaction was performed according to the protocol shown for KOD-plus polymerase, with 40 ng of pEF1/FGFR5 plasmid solution added as the template to a total volume of 50 µL of solution. Regarding reaction conditions, a cycle of treatment at 94°C for 1 minute, at 50°C for 1 minute, and at 68°C for 1 minute was repeated in 30 cycles. The DNA fragment obtained was purified using a QIAquick gel extraction kit, and subjected to terminal blunting with the Klenow fragment and re-purification using a QIAquick nucleotide removal kit, after which a second PCR was performed using the two DNA fragments obtained and primers 1 (forward) and 4 (reverse) (reaction conditions and the like were the same as the first PCR).

The PCR product obtained and pEF1/FGFR5 were respectively cleaved out using BamHI and ClaI and purified using a QIAquick gel extraction kit, whereby a FLAG inserted FGFR5 gene fragment and a plasmid fragment cleaved by BamHI and ClaI were obtained. The two kinds of DNA fragments obtained were subjected to a ligation reaction using a DNA ligation kit according to the protocol attached thereto, after which transformation into JM109 was performed. For the desired gene amplified by colony PCR, an endotoxin-free plasmid solution was prepared in a large amount using an Endofree Plasmid Maxi kit, and this plasmid was used as the FLAG inserted FGFR5 expression vector. Also, the vector obtained was confirmed as having the desired gene sequence by sequence analysis. The sequence analysis was performed according to a conventional method using the same primers as those used for constructing the FGFR5 expression vector.

### (2) Acquisition of constitutively FLAG-FGFR5-expressing CHO cells and preparation of antigen for immunization

(Materials)
reagent: GeneJammer Transfection reagent (STRATAGENE Ca#204130-21);
D-MEM (High glucose) (Nikken Ca#CM4402);
PBS(-) (Nikken Ca#CM6201);
FBS (CCT Ca#3008-502);
G418 (PAA Ca#P02-012);
Anti-FLAG antibody (Sigma);
anti-mouse Ig-PE-labeled secondary antibody
(Pharmingen BD);
30% BSA (Sigma)
device: FACSort(BD)

### (Method)

5 µg of the FLAG-FGFR5 expression vector prepared in (1) above was transfected into CHO cells seeded at 1 X 10⁶ to a 10-cm dish on the previous day, using the GeneJammer transfection reagent according to the manufacturer's instructions. On the day after the transfection, 3 mg/mL of G418 was added to the medium, selection with the drug was performed, and constitutively FLAG-FGFR5-expressing CHO cells were prepared.

3 X 10⁷ cells of the constitutively FLAG-FGFR5-expressing CHO cells obtained were washed with PBS(-), after which the cells were suspended and recovered from the dish by pipetting using a PBS(-) containing 5 mM EDTA and 0.5% BSA. After the recovery, the cells were washed using a PBS(-) containing 0.5% BSA, and the cells were suspended in a PBS(-) containing 0.5% BSA comprising 5 µg/mL of an anti-FLAG antibody, and an antibody reaction was performed at 4°C for 1 hour. After the reaction, the cells were twice washed using a PBS(-) containing 0.5% BSA, and an antibody reaction was performed at 4°C for 1 hour with an anti-mouse Ig-PE-labeled secondary antibody, diluted 400 fold with a PBS(-) containing 0.5% BSA. After the reaction, the cells were twice washed using a PBS(-) containing 0.5% BSA, and sorting of the cells was performed by FACSort. This sorting operation was twice repeated.

The highly FLAG-FGFR5-expressing CHO cells obtained above were cultured at 1.0 x 10⁶ cells/20 ml/15 cm dish using 100 dishes for 4 days, the cells were recovered using PBS(-)/10 mM EDTA, and the supernatant was removed via centrifugal operation, after which the cells were frozen with liquid nitrogen. The frozen cells were thawed on ice, suspended in a buffer solution containing a protease inhibitor (0.25 M Sucrose/10 mM MgCl₂/25 mM HEPES (pH 7.4)), and homogenized using a Potter homogenizer at 2000 rpm and 50 strokes. The cells were centrifuged at 1500 rpm and 4°C for 10 minutes, the supernatant was recovered, a fresh buffer solution was added to the precipitate, and the same operation was performed again. From the supernatant obtained by two times of homogenization, the precipitate was recovered via centrifugation at 50000 rpm and 4°C for 1 hour, and suspended in PBS(-) to obtain a cell membrane fraction, and this was used as the antigen for immunization.

### (3) Preparation of hybridoma and antibody

cell: PAI mouse myeloma
animal: Balb/c mouse 4-week-old
reagent: FCA Freund's Complete Adjuvant (CAPPEL Ca#55828);
FIA (Freund's Incomplete Adjuvant; CAPPEL Ca#55829);
PEG1500 (Roche, Ca#783641);
KBM medium (KOHJINBIO CO., Ca# 16009600);
1x HAT (COSMO BIO CO., LTD., 16213004);
1x HT (COSMO BIO CO., LTD., 16214004);
1x Penicillin-streptomycin-Glutamine (GIBCO BRL, Ca#10378-016);
10% FCS (PAA, Ultra Low IgG);
1% NEAA (GIBCO BRL, Ca#11140-050);
1% T-STIM (BD, Ca#354116)

### (Method)

A mouse was immunized with the cell membrane fraction obtained in (2) above using FCA for a first immunization, FIA for a second immunization, and the membrane fraction only for a third immunization at 50 µL per footpad. Two days after the third immunization, popliteal, inguinal and iliac lymph nodes of the mouse were recovered and washed, after which fusion with PAI cells was performed according to a conventional method. The fused cells were resuspended in KBM medium (Glutamine P/S, containing NEAA, T STIM, HAT and UL FBS) and seeded to a 96-well plate. After the cell fusion, the cells were cultured for about 10 days, whereby a culture supernatant containing an anti-FGFR-5 monoclonal antibody was obtained.

### (Example 11: Screening of anti-FGFR-5 monoclonal antibody based on affinity for FGFR-5)

### (1) Acquisition of constitutively FGFR5-expressing CHO cells for screening

(Materials)
reagent: pEF1/FGFR5;
FuGENE6 transfection reagent (Roche Ca#1-814-443);
Ham's F12 (GIBCO BRL Ca#11765-054);
PBS(-) (Nikken Ca#CM6201);
FBS (CCT Ca#3008-502);
G418 (PAA Ca#P02-012);
Anti-FLAG antibody (Sigma);
anti-mouse Ig-PE-labeled secondary antibody
(Pharmingen BD);
30% BSA (Sigma);
anti-FGFR5 monoclonal antibody R5B1-21
device: FACSort

### (Method)

6 µg of the FLAG-FGFR5 expression vector prepared in Example 10 (1) was transfected into CHO cells seeded at 1.2 X 10⁶ to a 10-cm dish on the previous day, using the FUGENE6 transfection reagent according to the manufacturer's instructions. On the day after the transfection, 3 mg/mL of G418 was added to the medium, selection with the drug was performed, and constitutively FGFR5-expressing CHO cells were prepared.

3 X 10⁷ cells of the constitutively FGFR5-expressing CHO cells obtained were washed with PBS(-), after which the cells were suspended and recovered from the dish by pipetting using a PBS(-) containing 5 mM EDTA and 0.5% BSA. After the recovery, the cells were washed using a PBS(-) containing 0.5% BSA, and the cells were suspended in a PBS(-) containing 0.5% BSA comprising 5 µg/mL of the anti-FGFR5 monoclonal antibody R5B1-21 (an antibody acquired according to Production Example 4, and previously confirmed to bind specifically to FGFR-5 using FGFR/Fc), and an antibody reaction was performed at 4°C for 1 hour. After the reaction, the cells were twice washed using a PBS(-) containing 0.5% BSA, and an antibody reaction was performed at 4°C for 1 hour with an anti-mouse Ig-PE-labeled secondary antibody diluted 400-fold with PBS(-) containing 0.5% BSA. After the reaction, the cells were twice washed using a PBS(-) containing 0.5% BSA, and sorting of the cells was performed by FACSort. This sorting operation was twice repeated to acquire constitutively FGFR5-expressing CHO cells, and these were used as the antigen-expressing cells for screening.

### (2) Screening method

(Materials)
reagent: Ham's F12 medium (GIBCO BRL Ca#11765-054);
PBS(-) (Nikken Ca#CM6201);
FBS (CCT Ca#3008-502);
G418 (PAA Ca#P02-012);
Anti-mouse Ig HRP (Amersham Ca#NA9310)
measurement device: Molecular Devices THERMO max micro plate reader

### (Method and results)

The constitutively FGFR5-expressing CHO cells acquired in (1) above and CHO cells incorporating an empty expression vector as the control were seeded to lanes B, D, F, and H, and lanes A, C, E, and G, respectively, of a 96-well plate, and cultured for 2 days or 3 days. After the medium was removed, a culture supernatant of the hybridoma obtained by cultivation after the cell fusion was applied to each well at 50 µL/well. After the application, the plate was incubated at room temperature for not less than 1 hour 30 minutes, and the culture supernatant was removed, after which the wells were twice washed using 1% FBS/PBS(-), and the anti-mouse Ig-HRP antibody (50 µL/well), diluted 2000 fold with the medium, was applied. Thereafter, the plate was incubated at room temperature for not less than 1 hour, and the secondary antibody was removed, after which the wells were washed using 1% FBS/PBS(-) 3 times. After the plate was washed, 50 µL/well of TMB reagent was added, and the reaction was allowed to proceed at room temperature for 10 minutes, after which 25 µL/well of 1N H₂SO₄ was added to stop the enzyme reaction. After the reaction was stopped, OD was measured at 450 nm.

After the above-described primary screening, a hybridoma that produces an antibody showing antigen reactivity was cloned with antigen reactivity as the index. From these results, six antibody clones having reactivity to FGFR5 (R5C7-10; R5D9-26; R5D10-23; R5D13-24; R5D14-19; R5D21-5) were selected.

### (Discussion)

As a result of this experiment, it was found that an assay system using cells having FGFR expressed forcedly therein could be used as an assay system for measuring the affinity of FGFR and a test compound.

### (Example 12: Analysis of site to which anti-FGFR-5 monoclonal antibody binds (epitope) in FGFR-5)

### (Outline)

Each Ig loop deficient FGFR5 expression vector was prepared, this was transiently expressed in CHO cells, and the reactivities of the antibodies obtained in Example 11 (R5C7-10; R5D9-26; R5D10-23; R5D13-24; R5D14-19; R5D21-5) to each Ig loop deficient FGFR5 were evaluated. Thereby the Ig loop with which each antibody reacts was identified. Also, the reactivities of the antibodies to the FLAG-inserted FGFR5 were evaluated at the same time; as a result, the reactivity to the non-FLAG-inserted FGFR5 was observed, but in some antibodies, the reactivity to the FLAG-inserted FGFR5 was lost. Hence, these antibodies were estimated to have reactivity to the vicinity of the FLAG insertion site; of these antibodies, those of high reactivity (R5D10-23, R5D14-19) and an antibody reactive to the 1st loop irrespective of whether or not FLAG was inserted (R5C7-10) were biotinylated, the capability of each antibody to inhibit the binding of the biotinylated antibody was evaluated, and epitopes were estimated.

### (Materials)

reagent: pEF1/FGFR5;
primer (JBioS);
(sequence on plasmid side)
1. TAA TAC GAC TCA CTA TAG GGA GAC CCA AGC TGG CTA GGT AAG CTT GGT A(SEQ ID NO: 10)
2. GCT TGG TAC CGA GCT CGG ATC CGC(SEQ ID NO: 11)
   (for B type preparation)
3. CTC CTT CCC TGG GCT AAT GTC ATC TCG GGC GGC GGC GGC CGG TGG GAA(SEQ ID NO: 12)
4. GAT GAC ATT AGC CCA GGG AAG GAG(SEQ ID NO: 13)
   (for C type preparation)
5. TCG TGC CCA CTG CTG GCT GGC GGG TCG GGC GGC GGC GGC CGG TGG GAA(SEQ ID NO: 14)
6. CCC GCC AGC CAG CAG TGG GCA CGA(SEQ ID NO: 15)
   (for D type preparation)
7. CAC CTT GTA GGT GGC GTT GAT GGC TCG GGC GGC GGC GGC CGG TGG GAA(SEQ ID NO: 16)
8. GCC ATC AAC GCC ACC TAC AAG GTG(SEQ ID NO: 17)
   (for B, C type FLAG insertion)
9. TTT ATC GTC ATC GTC TTT GTA GTC CCA CTG CTG GCT GGC GGG(SEQ ID NO: 18)
10. GAC TAC AAA GAC GAT GAC GAT AAA GCA CGA CCG CGC TTC ACA(SEQ ID NO: 19)
   (for D type FLAG insertion)
11. TTT ATC GTC ATC GTC TTT GTA GTC GAT CAC ATC CAC CTT GTA(SEQ ID NO: 20)
12. GAC TAC AAA GAC GAT GAC GAT AAA CAG CGG ACC CGT TCC AAG(SEQ ID NO: 21) ;
   Ham's F12 (GIBCO BRL Ca#11765-054);
   Penicillin/streptomycin (SIGMA Ca#103K2430);
   PBS(-) (Nikken Ca#CM6201);
   FBS (CCT Ca#3008-502);
   anti-mouse Ig-PE-labeled secondary antibody
   (Pharmingen BD);
   GeneJammer Transfection reagent (STRATAGENE
   Ca#204130-21);
   30% BSA (Sigma);
   Biotin labeling kit (Roche Diagnostics);
   strept-avidine-PE conjugated (Pharmingen BD)
   measurement device: FACSort

### (Method)

### (Construction of deficient FGFR5)

Three kinds of constructs were prepared, which were in a form lacking the 1st Ig loop (from the 27th glycine to the 117th leucine) from FGFR5 (SEQ ID NO:22 and 23) (B type), a form further lacking the 1st Ig loop to the acidic box (from the 27th glycine to the 138th aspartic acid) (C type), and a form lacking the 1st Ig loop to the 2nd Ig loop (from the 27th glycine to the 228th glycine) (D type). A first PCR reaction was performed using a combination of primers 1 and 3 and a combination of primers 2 and 4 for B type, a combination of primers 1 and 5 and a combination of primers 2 and 6 for C type, and a combination of primers 1 and 7 and a combination of primers 2 and 8 for D type. Regarding reaction conditions, a cycle of treatment at 94°C for 1 minute, at 55°C for 1 minute, and at 68°C for 1 minute was repeated in 35 cycles.

### (Construction of FLAG-inserted deficient FGFR5)

With each deficient FGFR5-expressing plasmid prepared as the template, FLAG insertion was performed. A FLAG tag was inserted between the 144th tryptophan and the 145th alanine for B type and C type, and between the 239th isoleucine and the 240th glutamine for D type (for the positions of the amino acids, see SEQ ID NO:22 and 23). A first PCR reaction was performed with a combination of primers 1 and 9 and a combination of primers 2 and 10 for B and C types, and a combination of primers 1 and 11 and a combination of primers 2 and 12 for D type. Regarding reaction conditions, a cycle of treatment at 94°C for 1 minute, at 55°C for 1 minute, and at 68°C for 1 minute was repeated in 35 cycles.

### (Transient overexpression in CHO cells)

1 µg of the prepared expression vector was transfected into CHO cells, seeded at 1.5 X 10⁵ cells/6 well dish on the previous day, using the GeneJammer transfection reagent according to the manufacturer's instructions.

### (Evaluation of antibody reactivity to transiently overexpressing CHO cells)

The CHO cells that transiently overexpress deficient and FLAG-inserted deficient FGFR5 obtained were washed with PBS(-), after which they were suspended and recovered from the dish by pipetting using a PBS(-) containing 5 mM EDTA and 0.5% BSA. After the recovery, the cells were washed using a PBS(-) containing 0.5% BSA, the cells were suspended in a PBS(-) containing 0.5% BSA comprising the anti-FGFR5 monoclonal antibody obtained (5 µg/mL), and an antibody reaction was allowed to proceed at 4°C for 1 hour. After the reaction, the cells were twice washed using a PBS(-) containing 0.5% BSA, and an antibody reaction was performed at 4°C for 1 hour with an anti-mouse Ig-PE-labeled secondary antibody, diluted 400 fold with a PBS(-) containing 0.5% BSA. After the reaction, the cells were twice washed using a PBS(-) containing 0.5% BSA, and the fluorescence intensity of the cells was evaluated by FACSort.

### (Biotinylation of antibody)

The antibody selected above was labeled with biotin according to the manufacturer's instructions.

### (Evaluation of inhibition of binding of biotinylated antibodies by non-labeled antibody)

Transiently FGFR5-expressing CHO cells were washed with PBS(-), after which the cells were suspended and recovered from the dish by pipetting using a PBS(-) containing 5 mM EDTA and 0.5% BSA. After the recovery, the cells were washed using a PBS(-) containing 0.5% BSA, and the cells were suspended in a PBS(-) containing 0.5% BSA comprising the anti-FGFR5 monoclonal antibody obtained (1 µg/mL), and an antibody reaction was performed at 4°C for 1 hour. After the reaction, the cells were twice washed using a PBS(-) containing 0.5% BSA, and a reaction was performed at 4°C for 1 hour with the biotinylated anti-FGFR5 antibody (5 µg/mL), diluted with a PBS(-) containing 0.5% BSA. After the reaction, the cells were twice washed using a PBS(-) containing 0.5% BSA, and an antibody reaction was performed at 4°C for 1 hour with strept-avidine-PE conjugated, diluted 800 fold with a PBS(-) containing 0.5% BSA. After the reaction, the cells were twice washed using a PBS(-) containing 0.5% BSA, and the fluorescence intensity of the cells was evaluated by FACSort.

### (Results)

The results are shown in Table 2 and Table 3.

**Table 2**

| Reactivity to deficient and FLAG-inserted deficient FGFR5-transiently expressing CHO cells | | | | | | | |
|---|---|---|---|---|---|---|---|
| | | R5C7-10 | R5D9-26 | R5D10-23 | R5D13-24 | R5D14-19 | R5D21-5 |
| A | FLAG (-) | + | + | + | + | + | + |
| | FLAG (+) | + | + | + | + | + | + |
| B | FLAG(-) | - | + | + | - | + | - |
| | FLAG(+) | - | - | - | - | - | - |
| C | FLAG(-) | - | + | + | - | + | - |
| | FLAG(+) | - | - | - | - | - | - |
| D | FLAG(-) | - | - | - | - | - | - |
| | FLAG(+) | - | - | - | - | - | - |

| | | | | | | | |
|---|---|---|---|---|---|---|---|
| +: reactive; -: not reactive | | | | | | | |

R5D10-23 and R5D14-19 that had lost the reactivity due to the insertion of FLAG were biotinylated as antibodies having reactivity to the vicinity of the FLAG insertion site. Also, as an antibody reactive to the 1^{st} loop, R5C7-10 was also biotinylated.

**Table 3**

| Evaluation of inhibition of binding of biotinylated antibodies by non-labeled antibody | | | |
|---|---|---|---|
| | biotinylation | | |
| | R5C7-10 | R5D10-23 | R5D14-19 |
| R5C7-10 | ++ | + | + |
| R5D9-26 | + | ++ | ++ |
| R5D10-23 | - | ++ | ++ |
| R5D13-24 | - | - | - |
| R5D14-19 | - | ++ | ++ |
| R5D21-5 | - | - | - |

| | | | |
|---|---|---|---|
| ++: inhibited + : weak inhibition - : no inhibition | | | |

By estimation of epitopes from the above-described results and based on the FLAG insertion site, it was assumed that an epitope of R5C7-10 was located on the 1st loop on the side near the FLAG insertion site; an epitope of R5D9-26 on the N-terminal side in the vicinity of the FLAG insertion site; an epitope of R5D10-23 on the C-terminal side from the R5D9-26 recognition site in the vicinity of the FLAG insertion site; an epitope of R5D13-24 on the N-terminal side farther from R5C7-10 on the 1st loop; an epitope of R5D14-19 on the C-terminal side from the R5D10-23 recognition site in the vicinity of the FLAG insertion site; and an epitope of R5D21-5 on the 1st loop (Fig. 12).

### (Example 13: Effects of anti-FGFR-5 monoclonal antibodies on the infectivity of a pseudotyped virus of HCV to Hep G2 cells)

### (Materials)

cell: HepG2
virus: ΔG/SEAP/HCV E1E2, ΔG/SEAP/VSV G
reagent: D-MEM high glucose(Nikken Bio Medical Laboratory), FBS (CTT), Reporter Assay Kit -SEAP- (TOYOBO SAK-101 Lot. 316100), anti-FGFR-5 monoclonal antibody R5C7-10, a nti-FGFR-5 monoclonal antibody R5D14-19

### (Method)

HepG2 was seeded at 8 x 10³ cells/well/80 µl/96-well plate, after which it was cultured (O/N) at 37°C in the presence of 5% CO₂, The anti-FGFR-5 antibodies R5C7-10 and R5D14-19 prepared according to Production Example 4 were prepared at a concentration 10 times the final concentration in PBS.

10 µl of each of the series of concentrations of R5C7-10, R5D14-19, and a mixture of R5C7-10 and R5D14-19 (individual concentrations therein are shown on the abscissa of Fig. 10 (C); total antibody concentrations are double the concentrations on the abscissa of Fig. 10 (C)) was added to the cells; after gentle stirring, the cells and respective antibodies were reacted at 37°C in the presence of 5% CO₂ for 1 hour. Also, for control, 10 µl of PBS was added to the cells, and the cells were allowed to stand at 37°C in the presence of 5% CO₂ for 1 hour. Thereafter, 1 x 10⁹ genome/10 µl of ΔG/SEAP/HCV E1E2 or 1 x 10⁷ genome/10 µl of ΔG/SEAP/VSV G was inoculated to each cell. These cells were cultured at 37°C in the presence of 5% CO₂ for 24 hours, after which the SEAP amount in each culture supernatant was measured according to the instructions of the Reporter Assay Kit, and the inhibition rate (%) for the cell infection of the virus by each concentration of FGFR-5/Fc was calculated with the SEAP amount for cells inoculated with the virus alone as the reference.

### (Results)

As a result of this experiment, as shown in Fig. 10, by the anti-FGFR-5 antibodies R5C7-10 and R5D14-19, the infection of ΔG/SEAP/HCV E1E2 to HepG2 cells was inhibited dose-dependently. R5C7-10 and R5D14-19 exhibited an infection inhibitory activity of up to about 50% when each was used alone; when these two antibodies were mixed, the mixture exhibited an infection inhibitory activity of up to about 70%. From this fact, it was suggested that at least some of compounds selected or judged as having affinity for FGFR by measuring the affinity for FGFR (measured using, for example, the method of Example 11 above) (for example, the antibodies obtained in Example 11 above) were compounds that inhibit the cell infection of HCV. Also, it was also suggested that a compound having affinity for FGFR or the capability of blocking the binding of FGFR and HCV (for example, antibodies against FGFR, particularly antibodies against FGFR-5, more particularly anti-FGFR-5 monoclonal antibodies) could be utilized as an inhibitor of the cell infection of HCV in the prevention or treatment of hepatitis C.

### (Example 14: Effects of anti-FGFR-5 monoclonal antibody on the infectivity of a pseudotyped virus of HCV to human hepatocyte primary culture)

### (Materials)

cell: human hepatocyte primary culture
virus: ΔG/SEAP/HCV E1E2, ΔG/SEAP/VSV G
reagent: D-MEM high glucose(Nikken Bio Medical Laboratory), FBS (CTT), Reporter Assay Kit -SEAP-(TOYOBO SAK-101 Lot. 316100), anti-FGFR-5 monoclonal antibody R5C7-10

### (Method)

Primary culture human hepatocytes were seeded at 8 x 10³ cells/well/80 µl/96 wp, after which they were cultured (O/N) at 37°C in the presence of 5% CO₂. The anti-FGFR-5 antibody R5C7-10 (R5C7-10) prepared according to Production Example 4 was prepared at a concentration 10 times the final concentration in PBS. Next, 10 µl of each of the series of concentrations of R5C7-10 prepared was added to the cells; after gentle mixing, the cells and the antibody were reacted at 37°C in the presence of 5% CO₂ for 1 hour. Also, for control, 10 µl of PBS was added to the cells, and the cells were allowed to stand at 37°C in the presence of 5% CO₂ for 1 hour. Thereafter, 1 x 10⁹ genome/10 µl of ΔG/SEAP/HCV E1E2 or 1 x 10⁷ genome/10 µl of ΔG/SEAP/VSV G was inoculated to each cell. These cells were cultured at 37°C in the presence of 5% CO₂ for 24 hours, after which the SEAP amount in each culture supernatant was measured according to the instructions of the Reporter Assay Kit, and the virus infection rate (%) for the cells treated with each concentration of FGFR-5/Fc was calculated with the SEAP amount for cells inoculated with the virus alone as the reference.

### (Results and discussion)

As a result of this experiment, as shown in Fig. 11, by the anti-FGFR-5 antibody R5C7-10, the infection of ΔG/SEAP/HCV E1E2 to human hepatocyte primary culture was inhibited dose-dependently. From this fact, it was suggested that at least some of compounds selected or judged as having affinity for FGFR by measuring the affinity for FGFR (measured using, for example, the method of Example 11 above) (for example, the antibodies obtained using the method of Example 11 above) were compounds that inhibit the cell infection of HCV. Also, it was also suggested that a compound having affinity for FGFR or the capability of blocking the binding of FGFR and HCV (for example, antibodies against FGFR, particularly antibodies against FGFR-5, more particularly anti-FGFR-5 monoclonal antibodies) could be utilized as an inhibitor of the cell infection of HCV in the prevention or treatment of hepatitis C.

### Industrial Applicability

The screening method and identification method of the present invention are useful for developing a pharmaceutical having novel action mechanism that inhibits the cell infection of HCV by inhibiting the interactions between fibroblast growth factor receptor (FGFR) and hepatitis C virus (HCV).

This application is based on patent application Nos. 2004-224741 and 2004-290106 filed in Japan, and US provisional patent application Nos. US60/605,298 and US60/618,238, the contents of which are incorporated in full herein by this reference.

SEQUENCE LISTING

<110>  JAPAN TOBACCO INC.

<120>  METHOD OF SCREENING COMPOUND PREVENTING CELLS FROM INFECTION WITH
HEPATITIS C VIRUS (HVC)

<130>  09792

<150>  JP2004-224741
<151>  2004-07-30

<150>  US 60/605,298
<151>  2004-08-27

<150>  JP2004-290106
<151>  2004-10-01

<150>  US 60/618,238
<151>  2004-10-13

<160>  23

<170>  PatentIn version 3.3

<210>  1

<211> 9548

<212> DNA

<213> Hepatitis C virus

<400> 1

gccagccccc tgatgggggc gacactccac catagatcac ccccctgtga ggaactactg 60

tcttcacgca gaaagcgtct agccatggcg ttagtatgag tgtcgtgcag cctccaggac 120

cccccctccc gggagagcca tagtggtctg cggaaccggt gagtacaccg gaattgccag 180

gacgaccggg tcctttcttg gatcaacccg ctcaatgcct ggagatttgg gcgtgccccc 240

gcgagactgc tagccgagta gtgttgggtc gcgaaaggcc ttgtggtact gcctgatagg 300

gtgcttgcga gtgccccggg aggtctcgta gaccgtgcat catgagcaca aatcctaaac 360

cccaaagaaa aaccaaacgt aacaccaacc gtcgcccaca ggacgttaag ttcccgggcg 420

gtggtcagat cgtcggtgga gtttacttgt tgccgcgcag gggccccagg ttgggtgtgc 480

gtgcgactag gaagacttcc gagcggtcgc aacctcgtgg aaggcgacaa cctatcccca 540

aggctcgccg gcccgagggc aggacctggg ctcagcctgg gtatccttgg cccctctatg 600

gcaatgaggg cttggggtgg gcaggatggc tcctgtcacc ccgcggctct cggcctagtt 660

ggggccctaa tgacccccgg cgtaggtcgc gtaatttggg taaggtcatc gataccctta 720

catgcggctt cgccgacctc atggggtaca tcccgcttgt cggcgccccc ttagggggcg 780

ctgccagggc cctggcacat ggtgtccggg ttctggagga cggcgtgaac tatgcaacag 840

ggaatttgcc cggttgctct ttctctatct tcctcttagc tctgctgtcc tgtttgacca 900

tcccagcttc cgcttatgaa gtgcgcaacg tgtccgggat ataccatgtc acaaacgact 960

gctccaactc aagcattgtg tatgaggcgg cggacgtgat catgcatgcc cccgggtgcg 1020

tgccctgcgt tcgggagaac aattcctccc gttgctgggt agcgctcact cccacgctcg 1080

cggccaggaa tgccagcgtc cccactacga cattacgacg ccacgtcgac ttgctcgttg 1140

ggacggctgc tttctgctcc gctatgtacg tgggggatct ctgcggatct gttttcctca 1200

tctcccagct gttcaccttc tcgcctcgcc ggcatgagac agtacaggac tgcaactgct 1260

caatctatcc cggccacgta tcaggccatc gtatggcttg ggatatgatg atgaactggt 1320

cgcccacggc agccttagtg gtgtcgcagt tactccggat cccacaagct gtcatggaca 1380

tggtggcggg ggcccactgg ggagtcctag cgggccttgc ctactattcc atggtgggga 1440

actgggctaa ggttttgatt gtgatgctac tctttgccgg cgttgacggg catacccgcg 1500

tgacgggggg ggtgcaaggc catgtcacct ctacactcac gtccctcttt agacctgggg 1560

cgtcccagaa aattcagctt gtaaacacca atggcagttg gcatatcaac aggactgccc 1620

tgaactgcaa tgactccctc aaaactgggt ttcttgccgc gctgttctac acacacaagt 1680

tcaacgcgtc cggatgcccg gagcgcatgg ccagctgtcg ctccattgac aagttcgacc 1740

agggatgggg tcccatcacc tatgctcaac ctgacaactc ggaccagagg ccgtattgct 1800

ggcactacgc acctcgacag tgtggtatcg tacccgcgtc gcaggtgtgc ggtccagtgt 1860

attgcttcac cccaagccct gttgtagtgg ggacgaccga tcgtttcggc gcccctacgt 1920

ataactgggg ggacaatgag acggacgtgc tgctcctaaa caacacgcgg ccgccgcatg 1980

gcaactggtt cggctgtaca tggatgaata gcactgggtt caccaagacg tgcggaggcc 2040

ccccgtgtaa catcaggggg gtcggcaaca acaccttgac ctgccccacg gactgcttcc 2100

ggaagcaccc cgacgccact tacacaaaat gtggttcggg cccttggttg acacctaggt 2160

gcttggttga ctacccatac aggctctggc actacccctg cactgtcaac tttaccatct 2220

tcaaggttag gatgtatgtg gggggcgtgg agcacaggct tgatgctgca tgcaactgga 2280

ctcgaggaga gcgttgcgac ttggaggaca gggatagagc agagctcagc ccgctattgc 2340

tgtctacaac agagtggcag atactgccct gttcctacac caccctaccg gctctgtcca 2400

ctggtttaat ccacctccac cagaacatcg tggacataca atacctgtac ggtatagggt 2460

cggcggtcgt ctccattgcc atcaagtggg agtatgtcgt gctgctcttc cttctcctgg 2520

cggacgcgcg cgtctgtgcc tgcttgtgga tgatgctgct gatagcccag gccgaggctg 2580

ccttagagaa cttggtggtc ctcaatgcgg cgtccgtggt cggagcgcat ggcatgctcc 2640

ccttctttat gttcttctgt gccgcctggt acatgaaggg caggctggtc cctggagcgg 2700

catacgcttt ctacggtgta tggccgctgc tcctgctcct gctagcatta ccaccacgag 2760

cttacgccat ggaccgggag atggttgcat cttgcggagg cggggttttt gtaggtctag 2820

cactcctgac cttgtcacca tactgtaaag tgttcctcgc taggctcata tggtggttac 2880

aatattttat caccaaagcc gaggcgcatt tgcaagtgtc gctccccccc ctcaacgttc 2940

gaggcggacg cgatgccatc atcctcctca tgtgcgcggt ccacccagag ctaatctttg 3000

acatcaccaa acttctgctc tccatactcg gtccgctcat ggtgctccaa gctagtttaa 3060

tccgagtgcc gtacttcgtg cgcgctcaag ggctcattcg cgcatgcatg ttggtgcgga 3120

aagctgccgg gggccattat gtccaaatgg ccttcgtgaa gctagctgcg ctgacaggca 3180

cgtacgttta tgaccacctc actccactgc aggattgggc ccatgtgggc ctacgagacc 3240

ttgcggtggc agtagagccc gttgtctttt ctgccatgga gaccaaggtc atcacctggg 3300

gggcagacac cgcggcgtgt ggggacatta tctcaggtct acccgtctcc gcccgaaggg 3360

ggaaggagat acttttggga ccggccgata gttttgaagg gcaggggtgg cgactccttg 3420

cgcccatcac ggcctactcc caacagacgc ggggcctact tggttgcatt atcactagtc 3480

tcacgggccg ggacaagaac caggttgagg gggaggttca agtggtctcc accgcaaaac 3540

aatctttcct ggcgacctgt gtcaacggcg cgtgctggac tgtctttcac ggtgcgggct 3600

caaagacctt agccgccgca aaaggcccaa tcacccaaat gtacaccaat gtagaccagg 3660

acctcgtcgg ctggccagcg ccccccgggg cgcgttcctt aacaccgtgc acctgcggca 3720

gctcggacct ttacctggtc acgagacatg ctgatgtcat cccggtgcgc cggcggggcg 3780

acagtagggg gagcttgctc tcccctagac ccatctccta cttgaagggc tcttcgggtg 3840

gtccattgct ctgcccctcg gggcacgttg tgggcatctt ccgggctgcc gtgtgcaccc 3900

ggggggtcgc gaaggcggtg gacttcatac ccgttgaatc catggaaacc actatgcggt 3960

ctccggtatt cacagacaac tcaacccccc cggccgtacc gcagaccttc caagtggccc 4020

atctacacgc ccccactggc agcggtaaaa gcactaaggt gccggctgca tatgcagccc 4080

aagggtacat ggtactcgtc ctgaacccgt ccgttgctgc caccctgggt tttggggcgt 4140

atatgtctaa ggcacatggc atcgacccca acatcagaac aggggtaagg accatcacca 4200

ccggcgcccc tatcacatac tccacctatg gcaagtttct tgccgatggt ggttgctctg 4260

gaggcgccta tgacatcata atatgtgatg agtgccactc aactgactcg acttccatcc 4320

tgggcatcgg cactgttttg gatcaagcgg agacggttgg agcgcggttc gtcgtgctcg 4380

ccaccgctac gcctccagga tcaatcactt ttccgcatcc caacatcgag gaggtgcccc 4440

tggccaacac aggggaaatc cccttctacg ccaaaaccat ccccattgag gtcatcaggg 4500

ggggaaggca cctcattttc tgccactcca aaaagaagtg tgacgagctc cccgcgaagc 4560

tgtcagccct cgggctcaac gctgtagcat attaccgggg gcttgacgtg tccgtcatac 4620

cggccagcgg agacgtcgtt gtcgtggcaa cagacgctct aatgacgggc tttaccggtg 4680

actttgattc agtgatcgac tgtaacacgt gtgtcaccca gacagtcgat tttagcttgg 4740

atcccacctt caccatcgag acgacgaccg tgccccaaga tgcggtttcg cgtacgcagc 4800

ggcgaggtag gactggtagg ggcaggagag gcatctacag gtttgtgact ccaggagaac 4860

ggccctcggc gatgttcgat tcttcggtcc tgtgtgagtg ttatgacgcg ggctgtgctt 4920

ggtatgagct cacgcccgct gagacctcgg ttaggttgcg ggcttaccta aatacaccag 4980

ggttgcccgt ctgccaggac catctggagt tctgggagag cgtcttcaca ggcctcaccc 5040

acatagacgc ccacttcttg tcccagacta agcaggcagg agacaacttc ccctacctgg 5100

tagcatacca agccacagtg tgcgccaggg ctaaggctcc acctccatcg tgggatcaaa 5160

tgtggaagtg tctcatacgg ctaaagccta cgctgcacgg gccaacgccc ctgctgtata 5220

ggctaggagc cgtccagaat gaggtcaccc tcacacaccc tataaccaaa tacatcatgg 5280

catgcatgtc ggctgaccta gaggtcgtca ctagcacttg ggtgctggta ggcggggtcc 5340

tcgcggctct ggccgcgtac tgcctgacaa cgggcagcgt ggtcattgtg ggcaggatca 5400

tcttgtccgg gaggccggcc gttattcccg acagggaagt tctctaccaa gagttcgatg 5460

aaatggaaga gtgcgcctcg cacctccctt acatcgaaca aggaatgcag ctcgccgagc 5520

aattcaagca gaaggcgctc ggtttgctgc aaacagccac caagcaagcg gaggctgctg 5580

ctcccgtggt ggagtccaag tggcgagccc ttgagacctt ctgggcgaag cacatgtgga 5640

atttcatcag cgggatacag tacttagcag gcttgtccac tctgcctgga aaccccgcaa 5700

tagcatcact gatggcattc acagcctcta tcaccagccc gctcgccacc caatataccc 5760

tcctgtttaa catcttgggg ggatgggtgg ccgcccaact cgcccccccc agtgccgctt 5820

cagccttcgt gggcgccggt atagctggcg cggctgttgg cagcataggc ctcgggaagg 5880

tgcttgtgga cattctggcg ggttatggag caggggtggc aggcgcgctc gtggccttta 5940

aggtcatgag cggtgacatg ccctccaccg aggacctggt caacttactc cccgccatcc 6000

tctctcctgg tgccctggtc gtcggggtcg tgtgcgcagc aatactgcgt cggcatgtgg 6060

gcccagggga gggggctgtg cagtggatga accggctgat agcgtttgct tcgcggggca 6120

accatgtctc ccccacgcac tatgtgcctg aaagcgacgc cgcagcgcgc gtcacccaga 6180

tcctctccaa ccttaccatc actcagctgt tgaagaggct tcaccagtgg attaatgagg 6240

actgctccac gccatgctcc ggctcgtggc tcagggatgt ttgggactgg atatgcacgg 6300

tattggctga tttcaagacc tggctccagt ccaagctcct gccgcggtta ccgggggtcc 6360

cttttttctc atgccagcgt gggtacaagg gggtttggcg gggagatggc atcatgtata 6420

ccacctgccc atgtggagca caaatcaccg gacatgtcaa aaacggttct atgaggatcg 6480

ttgggcctag aacctgtagc aacacgtggc acggaacatt tcccatcaac gcgtacacca 6540

caggcccctg cacaccctcc ccggcgccaa actattccag ggcgttgtgg cgggtggccg 6600

ctgaggagta tgtggaggtc acgcgggtgg gggatttcca ctacgtgacg ggcatgacca 6660

ctgacaacgt gaaatgccca tgccaggttc cggcccccga attcttcaca gaattggatg 6720

gggtgcggct gcacaggtac gctccggcgt gcaaacctct cctgcgggat gaggtcacat 6780

tccaggtcgg gctcaaccaa tatacggttg ggtcacagct cccatgtgag cccgaaccgg 6840

atgtaacagt ggtcacctcc atgctcaccg acccctccca cattacagca gaggcggcta 6900

ggcgtaggct ggccagaggg tctcccccctt ccttggccgg ttcttcagct agtcagttgt 6960

ctgcgctttc tttgaaggcg acatgcacta cccatcatgg cgccccagac actgacctca 7020

tcgaggccaa cctcctgtgg cggcaggaga tgggcggaaa catcacccgc gtggagtcag 7080

agaacaagat agtaattcta gactcttttg aaccgcttcg agcggaggag gatgagaggg 7140

aagtgtccgc tgcggcggag atcctgcgga agaccaggaa attccccgca gcgatgcccg 7200

tatgggcacg cccggactac aacccaccat tactagagtc ttggaagaac ccggactacg 7260

tccctccagt ggtacacggg tgcccattgc cgcctaccaa ggcccctcca ataccacctc 7320

cacggagaaa gagaacggtt gtcctgacag aatccaccgt gtcctctgcc ttggcggagc 7380

ttgctacaaa gacctttggc ggttccggat cgtcggccgt cgacagcggc acggcgaccg 7440

gccctcctga ccaggcctcc gccgaaggag atgcaggatc cgacgctgag tcgtactcct 7500

ccatgccccc ccttgaggga gagccggggg accccgatct cagcgacggg tcttggtcta 7560

ccgtaagcga ggaggccagc gaggacgtcg tctgctgctc gatgtcctac acatggacag 7620

gcgccttaat tacaccatgc gccgcggagg agagcaagct gcccattaat gcgctgagca 7680

accctttgct gcgccaccac aacatggtct attccacaac atcccgcagt gcaagcctgc 7740

ggcagaagaa ggtcaccttt gacagaatgc aagtcctgga cgatcattac cgggacgtgc 7800

tcaaggagat gaaggcgaag gcgtccacag tgaaggctaa actgctatct gtagaggagg 7860

catgcaagct gacgccccca cattcggcca aatccaaatt tggctatggg gcaaaggacg 7920

tccggagcct atccagcagg gccgttaacc acatccgctc cgtgtggaag gacttgctgg 7980

aggacactga cacaccaatt cagaccacca tcatggcaaa aaatgaggtt ttctgcgtcc 8040

aaccagagaa aggaggccgc aaaccagctc gccttatcgt attcccagac ctgggagttc 8100

gtgtgtgcga gaagatggcc ctttacgacg tggtttccac tcttcctcag gccgtgatgg 8160

gctcctcata cggattccag tactctccta agcagcgggt cgagttcctg gtgaatacct 8220

ggaaagcaaa gaagtgccct atgggctttt catatgacac ccgctgtttt gactcaacgg 8280

tcaccgagaa tgacatccgt gttgaggagt caatctacca atgttgtgac ttagcccccg 8340

aagctagaca ggccataagg tcgctcacag agcggctcta catcgggggt cccatgacta 8400

actccaaagg gcagaactgc ggctatcgcc ggtgccgcgc gagcggtgtg ctgacgacta 8460

gctgcggcaa taccctcaca tgttacttga aggccgctgc agcctgtcga gctgcaaagc 8520

tccaggactg cacgatgctt gtgtgcggag acgaccttgt cgttatctgt gatagcgcgg 8580

gaactcagga ggacgcggcg agcctacgag tcttcacgga ggctatgact aggtactctg 8640

cccccccggg ggacccgccc caaccagaat acgacttgga gctgataaca tcatgctcct 8700

ccaatgtgtc ggtcgcgcac gacgcatcag gcaaacgggt gtactatctc acccgtgacc 8760

ccaccacccc cctagcgcgg gctgcgtggg agacagctag acacactcca gtcaactcct 8820

ggctaggcaa catcatcatg tacgcgccca ccttatgggc aaggatgatt ctgatgaccc 8880

acttcttctc catccttcta gcccaggagc aacttgaaaa agccctagat tgtcagatct 8940

acggggccac ttactccatt gagccacttg acctacctca gatcattcaa cgactccacg 9000

gtcttagcgc attttcactc catagttact ctccaggtga gatcaatagg gtggcttcat 9060

gcctcaggaa acttggggta ccgcccttgc gagtctggag acatcgggcc agaagcgtcc 9120

gcgctaagct actgtcccag ggggggaggg ccgccacctg tggcaaatac ctcttcaact 9180

gggcagtaaa gaccaagctc aaactcactc caatcccaga agcgtcccag ctggacttgt 9240

ccggctggtt cgttgctggt tacagcgggg gagacatata tcacagcctg tctcgtgccc 9300

gaccccgctg gttcatgtgg tgcctactcc tactttccgt aggggtaggc atctacctgc 9360

tccccaaccg atgaacgggg agctaaacac tccaggccaa taggccatcc ccttcttttt 9420

tttttttttt tttttttcct tttcttcttt ggtggctcca tcttagccct agtcacggct 9480

agctgtgaaa ggtccgtgag ccgcatgact gcagagagtg ctgatactgg cctctctgca 9540

gatcatgt 9548

<210> 2

<211> 1134

<212> DNA

<213> Homo sapiens

<400> 2

atgacgccga gccccctgtt gctgctcctg ctgccgccgc tgctgctggg ggccttccca 60

ccggccgccg ccgcccgagg ccccccaaag atggcggaca aggtggtccc acggcaggtg 120

gcccggctgg gccgcactgt gcggctgcag tgcccagtgg agggggaccc gccgccgctg 180

accatgtgga ccaaggatgg ccgcaccatc cacagcggct ggagccgctt ccgcgtgctg 240

ccgcaggggc tgaaggtgaa gcaggtggag cgggaggatg ccggcgtgta cgtgtgcaag 300

gccaccaacg gcttcggcag cctgagcgtc aactacaccc tcgtcgtgct ggatgacatt 360

agcccaggga aggagagcct ggggcccgac agctcctctg ggggtcaaga ggaccccgcc 420

agccagcagt gggcacgacc gcgcttcaca cagccctcca agatgaggcg ccgggtgatc 480

gcacggcccg tgggtagctc cgtgcggctc aagtgcgtgg ccagcgggca ccctcggccc 540

gacatcacgt ggatgaagga cgaccaggcc ttgacgcgcc cagaggccgc tgagcccagg 600

aagaagaagt ggacactgag cctgaagaac ctgcggccgg aggacagcgg caaatacacc 660

tgccgcgtgt cgaaccgcgc gggcgccatc aacgccacct acaaggtgga tgtgatccag 720

cggacccgtt ccaagcccgt gctcacaggc acgcaccccg tgaacacgac ggtggacttc 780

ggggggacca cgtccttcca gtgcaaggtg cgcagcgacg tgaagccggt gatccagtgg 840

ctgaagcgcg tggagtacgg cgccgagggc cgccacaact ccaccatcga tgtgggcggc 900

cagaagtttg tggtgctgcc cacgggtgac gtgtggtcgc ggcccgacgg ctcctacctc 960

aataagctgc tcatcacccg tgcccgccag gacgatgcgg gcatgtacat ctgccttggc 1020

gccaacacca tgggctacag cttccgcagc gccttcctca ccgtgctgcc agacccaaaa 1080

ccgccagggc cacctgtggc ctcctcgtcc tcggccacta gcctgccgtg gccc 1134

<210> 3

<211> 675

<212> DNA

<213> Homo sapiens

<400> 3

actcacacat gcccaccgtg cccagcacca cctgtggcag gaccgtcagt cttcctcttc 60

cccccaaaac ccaaggacac cctcatgatc tcccggaccc ctgaggtcac gtgcgtggtg 120

gtggacgtga gccacgaaga ccccgaggtc cagttcaact ggtacgtgga cggcgtggag 180

gtgcataatg ccaagacaaa gccacgggag gagcagttca acagcacgtt ccgtgtggtc 240

agcgtcctca ccgttgtgca ccaggactgg ctgaacggca aggagtacaa gtgcaaggtc 300

tccaacaaag gcctcccagc ccccatcgag aaaaccatct ccaaaaccaa agggcagccc 360

cgagaaccac aggtgtacac cctgccccca tcccgggagg agatgaccaa gaaccaggtc 420

agcctgacct gcctggtcaa aggcttctac cccagcgaca tcgccgtgga gtgggagagc 480

aatgggcagc cggagaacaa ctacaagacc acacctccca tgctggactc cgacggctcc 540

ttcttcctct acagcaagct caccgtggac aagagcaggt ggcagcaggg gaacgtcttc 600

tcatgctccg tgatgcatga ggctctgcac aaccactaca cgcagaagag cctctccctg 660

tctccgggta aatga 675

<210> 4

<211> 24

<212> DNA

<213>  Artificial

<220>
<223>

<220>
<221>  CDS
<222>  (1)..(24)

<400>  4
ctc gag atc gaa ggt cgt ggt acc                                    24
Leu Glu Ile Glu Gly Arg Gly Thr
1               5

<210>  5
<211>  8
<212>  PRT
<213>  Artificial

<220>
<223>  Synthetic Construct

<400>  5

Leu Glu Ile Glu Gly Arg Gly Thr

1                5

<210>  6

<211>  49

<212>  DNA

<213>  Artificial

<220>

<223>  primer

<400>  6

taatacgact cactataggg agacccaagc tggctagtta agcttggta                49

<210>  7

<211>  49

<212>  DNA

<213>  Artificial

<220>

<223>  primer

<400>  7

tttatcgtca tcgtctttgt agtcctttgg ggggcctcgg gcggcggcg                49

<210> 8

<211> 49

<212> DNA

<213> Artificial

<220>

<223> primer

<400> 8

gactacaaag acgatgacga taaaatggcg gacaaggtgg tcccacggc 49

<210> 9

<211> 24

<212> DNA

<213> Artificial

<220>

<223> primer

<400> 9

gcttggtacc gagctcggat ccgc 24

<210> 10

<211> 49

<212> DNA

<213>  Artificial

<220>

<223>  primer

<400>  10

taatacgact cactataggg agacccaagc tggctaggta agcttggta                49

<210>  11

<211>  24

<212>  DNA

<213>  Artificial

<220>

<223>  primer

<400>  11

gcttggtacc gagctcggat ccgc                                           24

<210>  12

<211>  48

<212>  DNA

<213>  Artificial

<220>

<223>  primer

<400>  12
ctccttccct gggctaatgt catctcgggc ggcggcggcc ggtgggaa                48

<210>  13
<211>  24
<212>  DNA
<213>  Artificial

<220>
<223>  primer

<400>  13
gatgacatta gcccagggaa ggag                                          24

<210>  14
<211>  48
<212>  DNA
<213>  Artificial

<220>
<223>  primer

<400>  14

tcgtgcccac tgctggctgg cgggtcgggc ggcggcggcc ggtgggaa 48

<210> 15
<211> 24
<212> DNA
<213> Artificial

<220>
<223> primer

<400> 15
cccgccagcc agcagtgggc acga 24

<210> 16
<211> 48
<212> DNA
<213> Artificial

<220>
<223> primer

<400> 16
caccttgtag gtggcgttga tggctcgggc ggcggcggcc ggtgggaa 48

<210> 17

<211> 24

<212> DNA

<213> Artificial

<220>

<223> primer

<400> 17

gccatcaacg ccacctacaa ggtg 24

<210> 18

<211> 42

<212> DNA

<213> Artificial

<220>

<223> primer

<400> 18

tttatcgtca tcgtctttgt agtcccactg ctggctggcg gg 42

<210> 19

<211> 42

<212> DNA

<213>  Artificial

<220>

<223>  primer

<400>  19
gactacaaag acgatgacga taaagcacga ccgcgcttca ca                                42

<210>  20
<211>  42
<212>  DNA
<213>  Artificial

<220>

<223>  primer

<400>  20
tttatcgtca tcgtctttgt agtcgatcac atccaccttg ta                                42

<210>  21
<211>  42
<212>  DNA
<213>  Artificial

<220>

<223>  primer

<400>  21
gactacaaag acgatgacga taaacagcgg acccgttcca ag                    42

<210>  22
<211>  3105
<212>  DNA
<213>  Homo sapiens

<220>
<221>  CDS
<222>  (23)..(1537)

<400>  22
ccccaggtcc ggacaggccg ag atg acg ccg agc ccc ctg ttg ctg ctc ctg      52
                         Met Thr Pro Ser Pro Leu Leu Leu Leu Leu
                         1               5                   10

ctg ccg ccg ctg ctg ctg ggg gcc ttc ccg ccg gcc gcc gcc gcc cga      100
Leu Pro Pro Leu Leu Leu Gly Ala Phe Pro Pro Ala Ala Ala Ala Arg
                15                  20                  25

ggc ccc cca aag atg gcg gac aag gtg gtc cca cgg cag gtg gcc cgg      148
Gly Pro Pro Lys Met Ala Asp Lys Val Val Pro Arg Gln Val Ala Arg

            30                  35                  40

ctg ggc cgc act gtg cgg ctg cag tgc cca gtg gag ggg gac ccg ccg 196
Leu Gly Arg Thr Val Arg Leu Gln Cys Pro Val Glu Gly Asp Pro Pro
        45                  50                  55

ccg ctg acc atg tgg acc aag gat ggc cgc acc atc cac agc ggc tgg 244
Pro Leu Thr Met Trp Thr Lys Asp Gly Arg Thr Ile His Ser Gly Trp
    60                  65                  70

agc cgc ttc cgc gtg ctg ccg cag ggg ctg aag gtg aag cag gtg gag 292
Ser Arg Phe Arg Val Leu Pro Gln Gly Leu Lys Val Lys Gln Val Glu
75                  80                  85                  90

cgg gag gat gcc ggc gtg tac gtg tgc aag gcc acc aac ggc ttc ggc 340
Arg Glu Asp Ala Gly Val Tyr Val Cys Lys Ala Thr Asn Gly Phe Gly
                95                  100                 105

agc ctg agc gtc aac tac acc ctc gtc gtg ctg gat gac att agc cca 388
Ser Leu Ser Val Asn Tyr Thr Leu Val Val Leu Asp Asp Ile Ser Pro
            110                 115                 120

ggg aag gag agc ctg ggg ccc gac agc tcc tct ggg ggt caa gag gac 436
Gly Lys Glu Ser Leu Gly Pro Asp Ser Ser Ser Gly Gly Gln Glu Asp
        125                 130                 135

ccc gcc agc cag cag tgg gca cga ccg cgc ttc aca cag ccc tcc aag 484

Pro Ala Ser Gln Gln Trp Ala Arg Pro Arg Phe Thr Gln Pro Ser Lys
    140                 145                 150

atg agg cgc cgg gtg atc gca cgg ccc gtg ggt agc tcc gtg cgg ctc 532
Met Arg Arg Arg Val Ile Ala Arg Pro Val Gly Ser Ser Val Arg Leu
155                 160                 165                 170

aag tgc gtg gcc agc ggg cac cct cgg ccc gac atc acg tgg atg aag 580
Lys Cys Val Ala Ser Gly His Pro Arg Pro Asp Ile Thr Trp Met Lys
                175                 180                 185

gac gac cag gcc ttg acg cgc cca gag gcc gct gag ccc agg aag aag 628
Asp Asp Gln Ala Leu Thr Arg Pro Glu Ala Ala Glu Pro Arg Lys Lys
            190                 195                 200

aag tgg aca ctg agc ctg aag aac ctg cgg ccg gag gac agc ggc aaa 676
Lys Trp Thr Leu Ser Leu Lys Asn Leu Arg Pro Glu Asp Ser Gly Lys
        205                 210                 215

tac acc tgc cgc gtg tcg aac cgc gcg ggc gcc atc aac gcc acc tac 724
Tyr Thr Cys Arg Val Ser Asn Arg Ala Gly Ala Ile Asn Ala Thr Tyr
    220                 225                 230

aag gtg gat gtg atc cag cgg acc cgt tcc aag ccc gtg ctc aca ggc 772
Lys Val Asp Val Ile Gln Arg Thr Arg Ser Lys Pro Val Leu Thr Gly
235                 240                 245                 250

acg cac ccc gtg aac acg acg gtg gac ttc ggg ggg acc acg tcc ttc 820
Thr His Pro Val Asn Thr Thr Val Asp Phe Gly Gly Thr Thr Ser Phe
                255                 260                 265

cag tgc aag gtg cgc agc gac gtg aag ccg gtg atc cag tgg ctg aag 868
Gln Cys Lys Val Arg Ser Asp Val Lys Pro Val Ile Gln Trp Leu Lys
            270                 275                 280

cgc gtg gag tac ggc gcc gag ggc cgc cac aac tcc acc atc gat gtg 916
Arg Val Glu Tyr Gly Ala Glu Gly Arg His Asn Ser Thr Ile Asp Val
        285                 290                 295

ggc ggc cag aag ttt gtg gtg ctg ccc acg ggt gac gtg tgg tcg cgg 964
Gly Gly Gln Lys Phe Val Val Leu Pro Thr Gly Asp Val Trp Ser Arg
    300                 305                 310

ccc gac ggc tcc tac ctc aat aag ctg ctc atc acc cgt gcc cgc cag 1012
Pro Asp Gly Ser Tyr Leu Asn Lys Leu Leu Ile Thr Arg Ala Arg Gln
315                 320                 325                 330

gac gat gcg ggc atg tac atc tgc ctt ggc gcc aac acc atg ggc tac 1060
Asp Asp Ala Gly Met Tyr Ile Cys Leu Gly Ala Asn Thr Met Gly Tyr
                335                 340                 345

agc ttc cgc agc gcc ttc ctc acc gtg ctg cca gac cca aaa ccg cca 1108
Ser Phe Arg Ser Ala Phe Leu Thr Val Leu Pro Asp Pro Lys Pro Pro
            350                 355                 360

ggg cca cct gtg gcc tcc tcg tcc tcg gcc act agc ctg ccg tgg ccc 1156
Gly Pro Pro Val Ala Ser Ser Ser Ser Ala Thr Ser Leu Pro Trp Pro
        365                 370                 375

gtg gtc atc ggc atc cca gcc ggc gct gtc ttc atc ctg ggc acc ctg 1204
Val Val Ile Gly Ile Pro Ala Gly Ala Val Phe Ile Leu Gly Thr Leu
    380                 385                 390

ctc ctg tgg ctt tgc cag gcc cag aag aag ccg tgc acc ccc gcg cct 1252
Leu Leu Trp Leu Cys Gln Ala Gln Lys Lys Pro Cys Thr Pro Ala Pro
395                 400                 405                 410

gcc cct ccc ctg cct ggg cac cgc ccg ccg ggg acg gcc cgc gac cgc 1300
Ala Pro Pro Leu Pro Gly His Arg Pro Pro Gly Thr Ala Arg Asp Arg
                415                 420                 425

agc gga gac aag gac ctt ccc tcg ttg gcc gcc ctc agc gct ggc cct 1348
Ser Gly Asp Lys Asp Leu Pro Ser Leu Ala Ala Leu Ser Ala Gly Pro
            430                 435                 440

ggt gtg ggg ctg tgt gag gag cat ggg tct ccg gca gcc ccc cag cac 1396
Gly Val Gly Leu Cys Glu Glu His Gly Ser Pro Ala Ala Pro Gln His
        445                 450                 455

tta ctg ggc cca ggc cca gtt gct ggc cct aag ttg tac ccc aaa ctc 1444
Leu Leu Gly Pro Gly Pro Val Ala Gly Pro Lys Leu Tyr Pro Lys Leu

    460                 465                 470

tac aca gac atc cac aca cac aca cac aca cac tct cac aca cac tca 1492
Tyr Thr Asp Ile His Thr His Thr His Thr His Ser His Thr His Ser
475                 480                 485                 490

cac gtg gag ggc aag gtc cac cag cac atc cac tat cag tgc tag 1537
His Val Glu Gly Lys Val His Gln His Ile His Tyr Gln Cys
                495                 500

acggcaccgt atctgcagtg ggcacggggg ggccggccag acaggcagac tgggaggatg 1597

gaggacggag ctgcagacga aggcagggga cccatggcga ggaggaatgg ccagcacccc 1657

aggcagtctg tgtgtgaggc atagcccctg gacacacaca cacagacaca cacactgcct 1717

ggatgcatgt atgcacacac atgcgcgcac acgtgctccc tgaaggcaca cgtacgcaca 1777

cacgcacatg cacagatatg ccgcctgggc acacagataa gctgcccaaa tgcacgcaca 1837

cgcacagaga catgccagaa catacaagga catgctgcct gaacatacac acgcacaccc 1897

atgcgcagat gtgctgcctg gacacacaca cacacacgga tatgctgtct ggacgcacac 1957

acgtgcagat atggtatccg gacacacacg tgcacagata tgctgcctgg acacacagat 2017

aatgctgcct tgacacacac atgcacggat attgcctgga cacacacaca cacacgtgtg 2077

cacagatatg ctgtctggac acgcacacac atgcagatat gctgcctgga cacacacttc 2137

cagacacacg tgcacaggcg cagatatgct gcctggacac acgcagatat gctgtctagt 2197

cacacacaca cgcagacatg ctgtccggac acacacacgc atgcacagat atgctgtccg 2257

gacacacaca cgcacgcaga tatgctgcct ggacacacac acagataatg ctgcctcaac 2317

actcacacac gtgcagatat tgcctggaca cacacatgtg cacagatatg ctgtctggac 2377

atgcacacac gtgcagatat gctgtccgga tacacacgca cgcacacatg cagatatgct 2437

gcctgggcac acacttccgg acacacatgc acacacaggt gcagatatgc tgcctggaca 2497

cacgcagact gacgtgcttt tgggagggtg tgccgtgaag cctgcagtac gtgtgccgtg 2557

aggctcatag ttgatgaggg actttccctg ctccaccgtc actcccccaa ctctgcccgc 2617

ctctgtcccc gcctcagtcc ccgcctccat ccccgcctct gtcccctggc cttggcggct 2677

atttttgcca cctgccttgg gtgcccagga gtcccctact gctgtgggct ggggttgggg 2737

gcacagcagc cccaagcctg agaggctgga gcccatggct agtggctcat ccccactgca 2797

ttctccccct gacacagaga aggggccttg gtatttatat ttaagaaatg aagataatat 2857

taataatgat ggaaggaaga ctgggttgca gggactgtgg tctctcctgg ggcccgggac 2917

ccgcctggtc tttcagccat gctgatgacc acaccccgtc caggccagac accacccccc 2977

accccactgt cgtggtggcc ccagatctct gtaattttat gtagagtttg agctgaagcc 3037

ccgtatattt aatttatttt gttaaacatg aaagtgcatc ctttccctcc aaaaaaaaaa 3097

aaaaaaaa 3105

<210> 23

<211> 504

<212> PRT

<213> Homo sapiens

<400> 23

Met Thr Pro Ser Pro Leu Leu Leu Leu Leu Leu Pro Pro Leu Leu Leu

1               5                   10                  15

Gly Ala Phe Pro Pro Ala Ala Ala Ala Arg Gly Pro Pro Lys Met Ala

            20                  25                  30

Asp Lys Val Val Pro Arg Gln Val Ala Arg Leu Gly Arg Thr Val Arg

        35                  40                  45

Leu Gln Cys Pro Val Glu Gly Asp Pro Pro Pro Leu Thr Met Trp Thr
    50                  55                  60

Lys Asp Gly Arg Thr Ile His Ser Gly Trp Ser Arg Phe Arg Val Leu
65                  70                  75                  80

Pro Gln Gly Leu Lys Val Lys Gln Val Glu Arg Glu Asp Ala Gly Val
                85                  90                  95

Tyr Val Cys Lys Ala Thr Asn Gly Phe Gly Ser Leu Ser Val Asn Tyr
            100                 105                 110

Thr Leu Val Val Leu Asp Asp Ile Ser Pro Gly Lys Glu Ser Leu Gly
        115                 120                 125

Pro Asp Ser Ser Ser Gly Gly Gln Glu Asp Pro Ala Ser Gln Gln Trp
    130                 135                 140

Ala Arg Pro Arg Phe Thr Gln Pro Ser Lys Met Arg Arg Arg Val Ile
145                 150                 155                 160
Ala Arg Pro Val Gly Ser Ser Val Arg Leu Lys Cys Val Ala Ser Gly
                165                 170                 175
His Pro Arg Pro Asp Ile Thr Trp Met Lys Asp Asp Gln Ala Leu Thr
            180                 185                 190
Arg Pro Glu Ala Ala Glu Pro Arg Lys Lys Lys Trp Thr Leu Ser Leu
        195                 200                 205
Lys Asn Leu Arg Pro Glu Asp Ser Gly Lys Tyr Thr Cys Arg Val Ser
    210                 215                 220
Asn Arg Ala Gly Ala Ile Asn Ala Thr Tyr Lys Val Asp Val Ile Gln
225                 230                 235                 240
Arg Thr Arg Ser Lys Pro Val Leu Thr Gly Thr His Pro Val Asn Thr
                245                 250                 255

Thr Val Asp Phe Gly Gly Thr Thr Ser Phe Gln Cys Lys Val Arg Ser
            260                 265                 270

Asp Val Lys Pro Val Ile Gln Trp Leu Lys Arg Val Glu Tyr Gly Ala
        275                 280                 285

Glu Gly Arg His Asn Ser Thr Ile Asp Val Gly Gly Gln Lys Phe Val
    290                 295                 300

Val Leu Pro Thr Gly Asp Val Trp Ser Arg Pro Asp Gly Ser Tyr Leu
305                 310                 315                 320

Asn Lys Leu Leu Ile Thr Arg Ala Arg Gln Asp Asp Ala Gly Met Tyr
                325                 330                 335

Ile Cys Leu Gly Ala Asn Thr Met Gly Tyr Ser Phe Arg Ser Ala Phe
            340                 345                 350

Leu Thr Val Leu Pro Asp Pro Lys Pro Pro Gly Pro Pro Val Ala Ser
        355                 360                 365

Ser Ser Ser Ala Thr Ser Leu Pro Trp Pro Val Val Ile Gly Ile Pro
    370                 375                 380

Ala Gly Ala Val Phe Ile Leu Gly Thr Leu Leu Leu Trp Leu Cys Gln
385                 390                 395                 400

Ala Gln Lys Lys Pro Cys Thr Pro Ala Pro Ala Pro Pro Leu Pro Gly
                405                 410                 415

His Arg Pro Pro Gly Thr Ala Arg Asp Arg Ser Gly Asp Lys Asp Leu
            420                 425                 430

Pro Ser Leu Ala Ala Leu Ser Ala Gly Pro Gly Val Gly Leu Cys Glu
        435                 440                 445

Glu His Gly Ser Pro Ala Ala Pro Gln His Leu Leu Gly Pro Gly Pro
    450                 455                 460

Vàl Ala Gly Pro Lys Leu Tyr Pro Lys Leu Tyr Thr Asp Ile His Thr

465                 470                 475                 480

His Thr His Thr His Ser His Thr His Ser His Val Glu Gly Lys Val

                485                 490                 495

His Gln His Ile His Tyr Gln Cys

            500

## Claims

1. A screening method for a compound inhibiting cell infection of hepatitis C virus (HCV), which comprises
(a) a step of measuring an affinity of a test compound and fibroblast growth factor receptor (FGFR) having a binding activity to a surface protein of HCV and/or a capability of cell incorporation of HCV; and
(b) a step of selecting the test compound having the affinity.

2. The method of claim 1, further comprising
(c) a step of measuring the degree of cell infection of HCV in the presence and absence of the selected test compound; and
(d) a step of comparing both the measured values.

3. The method of claim 2, wherein the degree of cell infection of HCV is measured using a pseudotyped virus of HCV.

4. The method of any one of claims 1 to 3, wherein said FGFR is FGFR-4.

5. The method of any one of claims 1 to 3, wherein said FGFR is FGFR-5.

6. The method of any one of claims 1 to 5, wherein a membrane fraction of a cell having FGFR is used.

7. The method of any one of claims 1 to 5, wherein a cell having FGFR expressed forcedly therein is used.

8. The method of any one of claims 1 to 5, wherein a solubilized FGFR is used.

9. The method of any one of claims 1 to 5, wherein said affinity is a binding activity between the test compound and an FGF-binding site of FGFR.

10. A screening method for a compound inhibiting a cell infection of hepatitis C virus (HCV), which comprises
(a) a step of measuring a capability of a test compound to block binding of HCV and fibroblast growth factor receptor (FGFR) having a binding activity to a surface protein of HCV; and
(b) a step of selecting the test compound having the capability of blocking.

11. The method of claim 10, further comprising
(c) a step of measuring the degree of cell infection of HCV in the presence and absence of the selected test compound; and
(d) a step of comparing both the measured values.

12. The method of claim 11, wherein the degree of cell infection of HCV is measured using a pseudotyped virus of HCV.

13. The method of any one of claims 10 to 12, wherein said FGFR is FGFR-4.

14. The method of any one of claims 10 to 12, wherein said FGFR is FGFR-5.

15. The method of any one of claims 10 to 14, wherein a membrane fraction of a cell having FGFR is used.

16. The method of any one of claims 10 to 14, wherein a cell having FGFR expressed forcedly therein is used.

17. The method of any one of claims 10 to 14, wherein a solubilized FGFR is used.

18. The method of any one of claims 10 to 14, wherein said capability of blocking is a capability of the test compound to block the binding of HCV and an FGF-binding site of FGFR.

19. A screening kit for a compound inhibiting cell infection of HCV, which comprises a lipid bilayer membrane containing FGFR and/or a FGFR-expressing cell, and a pseudotyped virus of HCV.

20. A method of identifying a compound inhibiting cell infection of hepatitis C virus (HCV), which comprises
(a) a step of measuring an affinity of a test compound and fibroblast growth factor receptor (FGFR) having a binding activity to a surface protein of HCV and/or a capability of cell incorporation of HCV; and
(b) a step of judging the test compound having the affinity.

21. The method of claim 20, further comprising
(c) a step of measuring the degree of cell infection of HCV in the presence and absence of the judged test compound; and
(d) a step of comparing both the measured values.

22. The method of claim 21, wherein the degree of cell infection of HCV is measured using a pseudotyped virus of HCV.

23. The method of any one of claims 20 to 22, wherein said FGFR is FGFR-4.

24. The method of any one of claims 20 to 22, wherein said FGFR is FGFR-5.

25. The method of any one of claims 20 to 24, wherein a membrane fraction of a cell having FGFR is used.

26. The method of any one of claims 20 to 24, wherein a cell having FGFR expressed forcedly therein is used.

27. The method of any one of claims 20 to 24, wherein a solubilized FGFR is used.

28. The method of any one of claims 20 to 24, wherein said affinity is a binding activity between the test compound and an FGF-binding site of FGFR.

29. A method of identifying a compound inhibiting a cell infection of hepatitis C virus (HCV), which comprises
(a) a step of measuring a capability of a test compound to block the binding of HCV and fibroblast growth factor receptor (FGFR) having a binding activity to a surface protein of HCV; and
(b) a step of judging the test compound having the capability of blocking.

30. The method of claim 29, further comprising
(c) a step of measuring the degree of cell infection of HCV in the presence and absence of the judged test compound; and
(d) a step of comparing both the measured values.

31. The method of claim 30, wherein the degree of cell infection of HCV is measured using a pseudotyped virus of HCV.

32. The method of any one of claims 29 to 31, wherein said FGFR is FGFR-4.

33. The method of any one of claims 29 to 31, wherein said FGFR is FGFR-5.

34. The method of any one of claims 29 to 33, wherein a membrane fraction of a cell having FGFR is used.

35. The method of any one of claims 29 to 33, wherein a cell having FGFR expressed forcedly therein is used.

36. The method of any one of claims 29 to 33, wherein a solubilized FGFR is used.

37. The method of any one of claims 29 to 33, wherein said capability of blocking is a capability of the test compound to block the binding of HCV and an FGF-binding site of FGFR.

38. A kit for identifying a compound inhibiting cell infection of HCV, which comprises a lipid bilayer membrane containing FGFR and/or a cell expressing FGFR, and a pseudotyped virus of HCV.

39. An inhibitor of cell infection of HCV, which comprises a compound having an affinity for FGFR having a binding activity to a surface protein of HCV and/or a capability of cell incorporation of HCV.

40. The inhibitor of claim 39, wherein said FGFR is FGFR-4.

41. The inhibitor of claim 39, wherein said FGFR is FGFR-5.

42. The inhibitor of any one of claims 39 to 41, wherein said compound is a low molecular compound.

43. The inhibitor of any one of claims 39 to 41, wherein said compound is a ligand for FGFR.

44. The inhibitor of claim 43, wherein said ligand for FGFR is FGF or a fragment thereof.

45. The inhibitor of claim 43, wherein said ligand for FGFR is an antibody against FGFR-4.

46. The inhibitor of claim 43, wherein said ligand for FGFR is an antibody against FGFR-5.

47. The inhibitor of any one of claims 39 to 41, wherein said affinity is a binding activity between an FGF-binding site of FGFR and a compound.

48. An inhibitor of cell infection of HCV, which comprises a solubilized FGFR based on FGFR having a binding activity to a surface protein of HCV and/or a capability of cell incorporation of HCV.

49. The inhibitor of cell infection of HCV of claim 48, wherein said solubilized FGFR is a solubilized FGFR-4.

50. The inhibitor of cell infection of HCV of claim 48, wherein said solubilized FGFR is a solubilized FGFR-5.

51. An inhibitor of cell infection of HCV, comprising a compound having a capability of blocking the binding of HCV and FGFR having a binding activity to a surface protein of HCV.

52. The inhibitor of claim 51, wherein said FGFR is FGFR-4.

53. The inhibitor of claim 51, wherein said FGFR is FGFR-5.

54. The inhibitor of any one of claims 51 to 53, wherein said compound is a low molecular compound.

55. The inhibitor of any one of claims 51 to 53, wherein said compound is a ligand for FGFR.

56. The inhibitor of claim 55, wherein said ligand for FGFR is FGF or a fragment thereof.

57. The inhibitor of claim 55, wherein said ligand for FGFR is an antibody against FGFR-4.

58. The inhibitor of claim 55, wherein said ligand for FGFR is an antibody against FGFR-5.

59. The inhibitor of any one of claims 51 to 53, wherein said compound is a solubilized FGFR.

60. The inhibitor of claim 59, wherein said solubilized FGFR is a solubilized FGFR-4.

61. The inhibitor of claim 59, wherein said solubilized FGFR is a solubilized FGFR-5.

62. The inhibitor of any one of claims 51 to 53, wherein said capability of blocking is a capability of the test compound to block the binding of HCV and an FGF-binding site of FGFR.

63. A method of inhibiting cell infection of HCV, which comprises administering, to a patient in need of inhibition of cell infection of HCV, a therapeutically effective amount of a compound having an affinity for an FGFR having a binding activity to a surface protein of HCV and/or a capability of cell incorporation of HCV.

64. A method of inhibiting cell infection of HCV, which comprises administering, to a patient in need of inhibition of cell infection of HCV, a therapeutically effective amount of a solubilized FGFR based on FGFR having a binding activity to a surface protein of HCV and/or a capability of cell incorporation of HCV.

65. A method of inhibiting cell infection of HCV, which comprises administering, to a patient in need of inhibition of cell infection of HCV, a therapeutically effective amount of a compound having a capability of blocking the binding of HCV and FGFR having a binding activity to a surface protein of HCV.

66. Use of a compound having an affinity for an FGFR having a binding activity to a surface protein of HCV and/or a capability of cell incorporation of HCV, for the manufacture of an inhibitor of cell infection of HCV.

67. Use of a solubilized FGFR based on an FGFR having a binding activity to a surface protein of HCV and/or a capability of cell incorporation of HCV, for the manufacture of an inhibitor of cell infection of HCV.

68. Use of a compound having a capability of blocking the binding of an FGFR having a binding activity to a surface protein of HCV and HCV, for the manufacture of an inhibitor of cell infection of HCV.
